(19) Europäisches Patentamt / European Patent Office / Office européen des brevets

(11) **EP 3 449 009 B1**

(12) **EUROPEAN PATENT SPECIFICATION**

(45) Date of publication and mention
of the grant of the patent:
**18.08.2021 Bulletin 2021/33**

(21) Application number: **17721576.1**

(22) Date of filing: **25.04.2017**

(51) Int Cl.:
***C12Q 1/68*** *(2018.01)*

(86) International application number:
**PCT/EP2017/059800**

(87) International publication number:
**WO 2017/186719 (02.11.2017 Gazette 2017/44)**

(54) **MICRORNA BIOMARKERS IN BLOOD FOR DIAGNOSIS OF ALZHEIMER'S DISEASE**

MICRORNA-BIOMARKER IN BLUT ZUR DIAGNOSE VON MORBUS ALZHEIMER

MICRO-ARN BIOMARQUEURS DANS LE SANG DESTINÉ AU DIAGNOSTIC DE LA MALADIE D'ALZHEIMER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2016 PL 41695616
29.04.2016 PCT/IB2016/052440**

(43) Date of publication of application:
**06.03.2019 Bulletin 2019/10**

(73) Proprietors:
• **Instytut Biologii Doswiadczalnej
PAN. im. M. Nenckiego
02-093 Warszawa (PL)**
• **Biotech Innovations Spólka Z Ograniczona
Odpowiedzialnoscia w likwidacji
02-109 Warszawa (PL)**

(72) Inventors:
• **WOJDA, Urszula
02-104 Warszawa (PL)**
• **LASKOWSKA-KASZUB, Katarzyna
00-131 Warszawa (PL)**
• **GABRYELEWICZ, Tomasz
00-815 Warszawa (PL)**
• **KUZNICKI, Jacek
02-202 Warszawa (PL)**

(74) Representative: **Patpol Kancelaria Patentowa Sp. z o.o.
Nowoursynowska 162J
02-776 Warszawa (PL)**

(56) References cited:
**EP-A1- 2 733 220        WO-A1-2012/145363
WO-A1-2013/024469        WO-A2-2013/003350
WO-A2-2013/022953        WO-A2-2015/095862**

• **HIROSHA GEEKIYANAGE ET AL: "Blood serum miRNA: Non-invasive biomarkers for Alzheimer's disease", EXPERIMENTAL NEUROLOGY, vol. 235, no. 2, 1 June 2012 (2012-06-01), pages 491-496, XP055058220, ISSN: 0014-4886, DOI: 10.1016/j.expneurol.2011.11.026**
• **SHI LIN ET AL: "miR-483-5p and miR-486-5p are down-regulated in cumulus cells of metaphase II oocytes from women with polycystic ovary syndrome", REPRODUCTIVE BIOMEDICINE ONLINE, vol. 31, no. 4, 1 October 2015 (2015-10-01), pages 565-572, XP029286523, ISSN: 1472-6483, DOI: 10.1016/J.RBMO.2015.06.023**

EP 3 449 009 B1

**Description**

**TECHNICAL FIELD**

The present invention provides a method of *ex vivo* diagnosing Alzheimer's disease and/or an early stage of Alzheimer's disease in a subject.

**BACKGROUND ART**

The most common cause of age-related dementia worldwide is Alzheimer's disease (AD), a neurodegenerative disorder that progressively and irreversibly impairs memory and cognition, and eventually leads to a massive loss of brain neurons and death. With a worldwide ageing trend among populations, the number of AD cases is rapidly growing. As of 2013, there were an estimated 44.4 million people with dementia globally. This number will increase to an estimated 75.6 million in 2030 [1]. The global costs related to AD are similar to the financial burden of heart disease and cancer, placing AD among the major unmet health concerns [2]. Despite intense research worldwide, no effective treatments for AD exist because its cause(s) remain unclear. One of the main reasons is the lack of readily available biomarkers and tests that make the early and reliable diagnosis of AD possible, although mounting evidence indicates that the effectiveness of therapeutic modalities critically depends on the early diagnosis of AD, before massive neuron loss occurs.

Advances in our knowledge of AD have shown that clinical AD symptoms usually develop after a preclinical pathogenic process several decades long, defined as the asymptomatic preclinical stage [3]. This stage is followed by the early clinical AD stage, which occurs in some patients with Mild Cognitive Impairment (MCI). Memory problems in MCI may be minimal to mild but in time MCI patients convert to AD at a rate of approximately 10% to 15% per year [4, 5]. After early stage, clinical AD progresses from mild through moderate to severe, late stage. Advances in our understanding of the disease stages are reflected in the recently updated diagnostic and research criteria for AD [6-8]. There are no tools sensitive enough to diagnose preclinical AD. The updated core clinical diagnostic criteria allow only for the diagnosis of probable or possible AD in patients with clinical dementia and in MCI patients (MCI attributable to AD; early AD). A definite AD diagnosis is presently possible only based on the *post* mortem histopathological examination of the brain.

In current clinical practice, the diagnosis of AD starts with neuropsychological testing such as the Mini-Mental State Examination (MMSE). However, neuropsychological assays cannot, for example, detect early AD in patients with Subjective Cognitive Impairment (SCI) who solve the tests with high scores due to their generally high cognitive abilities, but who subjectively observe a decline in their mental efficiency. Currently, AD diagnostics can be supported by a cerebrospinal fluid (CSF) biochemical assay of "total" and hyperphosphorylated tau protein (increased CSF levels), and the 42-amino-acid isoform of the $A\beta$ peptide (decreased CSF levels), reflective of brain pathology. Brain imaging procedures such as positron emission tomography (PET) and structural magnetic resonance imaging (sMRI) can further support the diagnosis. All of these AD assays have significant cost and access-to-care barriers. The enormously high costs and requirements for sophisticated equipment represent a fundamental barrier for the application of brain imaging as a large-scale AD screening tool in most clinical settings. Although CSF seems to reflect the biochemical changes occurring in the brain and, therefore, is considered the most suitable biological fluid to study neurodegenerative diseases, CSF assays also involve high costs, require a lumbar puncture which is an invasive, uncomfortable and relatively risky procedure. Moreover, they are not always feasible, especially in the elderly. Because of limits involved in frequent, multiple lumbar punctures, CSF assays are also not suitable for the concurrent monitoring of therapeutic trials, drug efficacy, and for longitudinal studies. Therefore, while the recent development of technologies addressing the 'AD signature' in CSF reflects significant progress in AD diagnosis, CSF biomarkers do not and are not likely to fulfill optimal diagnostic criteria for clinical practice.

As indicated by the Food and Drug Administration (FDA), a preferable biomarker for clinical applications should be available in biological samples that are easy to obtain in a safe, non-invasive procedure, and the laboratory methods must be reliable, stable, and cost-effective. In light of the preferable biomarker criteria, it becomes clear that progress in AD diagnostics relies to a great extent on the identification of novel diagnostic AD biomarkers of improved sensitivity and specificity, in more easily available diagnostic tissues, such as blood [8, 9]. Accordingly, exponentially growing research efforts have recently focused on the development and validation of non-invasive and generalizable blood-based biomarkers. All systemic biology paradigms have been utilized as modalities in the search for blood-based AD molecular signatures, including proteomics, lipidomics, transcriptomics, metabolomics, and epigenetics [9]. However, this research faces multiple obstacles and at present no defined test based on blood biomarkers has been approved for clinical practice.

One of the most promising approaches to the identification of blood-based AD biomarkers concentrates on circulating microRNAs (miRNAs) [9-11]. MicroRNAs represent one class of small noncoding RNA molecules, approximately 22 bp in length, which mainly repress gene expression at the post-transcriptional level by binding and inhibiting particular mRNA targets. So far, over 2000 miRNAs have been identified in the human genome and it appears that >60% of human protein-coding genes are regulated by miRNAs. The study of the possible application of miRNAs as AD biomarkers was prompted by the increasing evidence of the significant regulatory functions of miRNA in different pathologies including neurodegeneration, and the altered expression of miRNAs reported in many disease states in biofluids [12-14]. miRNAs are abundant in the blood stream and can operate both in the adjacent cells as well as in more distant areas of the body via a mechanism similar to hormones [13-15]. miRNAs have been reported to be transported in blood in exosomes, high-density lipoproteins, and in complexes with proteins such as Argonaute2, protecting them from degradation [16-19]. Database entries based on circulating miRNAs have increased dramatically in recent years, which suggests that the identification of circulating miRNAs as disease biomarkers is one of the hottest topics in miRNA research [12]. However, despite several investigations, so far no miRNA AD biomarkers have been approved.

Some prior art discloses the use of detecting miRNAs to diagnose AD [9-11, 20-30] but no prior art disclosed a method based on the miRNA signatures reported in this application. Moreover, no prior art has hitherto disclosed a panel of blood plasma-based miRNAs, which were determined in AD and MCI patients diagnosed both by neuropsychological tests and CSF (cerebrospinal fluid) biomarkers (miRNAs biomarkers identified in relation with the CSF biomarkers), and moreover, adhering with the current guidelines for the standardization of pre-analytic variables for blood-based biomarkers [31]. Moreover, no prior art reports miRNAs that can detect early AD in SCI patients. Moreover, no prior art reports miRNAs that can differentiate early from later AD stages.

Up to now, the search for reliable miRNA biomarkers for AD in peripheral blood has been very challenging because of difficulties with the standardization of the analytical methods and the low reproducibility of the results. The lack of success in the identification of adequate and reproducible blood-based miRNA biomarkers is currently seen as, to a high degree, the result of substantial variability across various diagnostic centers in pre-analytical, analytical and post-analytical factors, referring to both the diagnoses of patients recruited for studies and to the methodology of blood handling and miRNA analysis [31, 32]. To respond to the necessity for extensive standardization of materials and procedures, in their study, the inventors employed current guidelines for the standardization of pre-analytic variables for blood-based biomarkers [31]. Moreover, the selection of study subjects involved diagnoses of AD based on neuropsychological tests and neurological examination in adherence to the current golden standards [6, 7], as well as based on CSF markers, which were determined both in MCI, SCI and AD patients according to current recommendations and using recommended by European JPND BIOMARKAPD consortium standardized CSF assays [31]. Only two previously reported studies of circulating miRNA in AD were performed in AD patients in which neuropsychological tests results were confirmed by other biochemical tests, in particularly by biochemical CSF assay [23, 29]. None of previously reported studies was performed in MCI patients in which CSF assay was performed and indicated early AD stage.

## DISCLOSURE OF THE INVENTION

The invention is defined by the appended claims. Disclosed is a panel of biomarker microRNAs for use in a method of diagnosing Alzheimer's disease (AD) based on a profile of specific microRNAs that are present in the blood. More specifically, the panel can be used to detect an early stage of AD in subjects with Mild Cognitive Impairment (MCI) due to Alzheimer's disease or with Subjective Cognitive Impairment due to Alzheimer's disease and to diagnose AD in patients with clinical dementia.

Disclosed is a panel of biomarker microRNAs for use in a method of diagnosing Alzheimer's disease and/or Mild Cognitive Impairment (MCI) due to Alzheimer's disease and/or Subjective Cognitive Impairment (SCI) due to Alzheimer's disease, in particular for detection of an early stage of Alzheimer's disease in subjects with Mild Cognitive Impairment or with Subjective Cognitive Impairment and/or for diagnosis of Alzheimer's disease in patients with clinical dementia in a subject, wherein the panel comprises at least one selected from hsa-miR-151a-5p (SEQ ID NO: 1), hsa-miR-30b-5p (SEQ ID NO: 2), hsa-miR-486-5p (SEQ ID NO: 3), hsa-miR-33a-5p (SEQ ID NO: 4), hsa-miR-483-5p (SEQ ID NO: 5), hsa-miR-18a-5p (SEQ ID NO: 6), hsa-miR-320a (SEQ ID NO: 7), hsa-miR-320b (SEQ ID NO: 8), hsa-miR-320c (SEQ ID NO: 9) and the method involves assessing the level of said biomarker microRNA(s) in subject blood sample.

The panel may comprise at least one additional biomarker microRNA(s) selected from the group consisting of hsa-miR-502-3p (SEQ ID NO 14), hsa-miR-103a-3p (SEQ ID NO: 15), hsa-miR-301a-3p (SEQ ID NO: 16), hsa-miR-142-3p (SEQ ID NO: 17), hsa-miR-200a-3p (SEQ ID NO: 18), hsa-miR-1260a (SEQ ID NO: 19).

The panel may comprise at least one of hsa-miR-483-5p (SEQ ID NO: 5) and/or hsa-miR-486-5p (SEQ ID NO: 3).

Thus, disclosed is a panel of biomarker microRNAs comprising hsa-miR-483-5p (SEQ ID NO: 5) and hsa-miR-486-5p (SEQ ID NO: 3) for the uses disclosed herein, e.g., for uses in a method of diagnosing Alzheimer's disease and/or Mild Cognitive Impairment (MCI) due to Alzheimer's disease and/or Subjective Cognitive Impairment (SCI) due to Alzheimer's disease, in particular for the detection of an early stage of Alzheimer's disease in subjects with Mild Cognitive Impairment or with Subjective Cognitive Impairment and/or for diagnosis of Alzheimer's disease in patients with clinical dementia in a subject, and wherein the method involves assessing the level of said biomarker microRNA(s) in subject blood sample.

The panel may comprise at least two, preferably three, preferably four, preferably five, preferably six, preferably seven, preferably eight, preferably all nine biomarker microRNAs selected from hsa-miR-151a-5p (SEQ ID NO: 1), hsa-miR-30b-5p (SEQ ID NO: 2), hsa-miR-486-5p (SEQ ID NO: 3), hsa-miR-33a-5p (SEQ ID NO: 4), hsa-miR-483-5p (SEQ ID NO: 5), hsa-miR-18a-5p (SEQ ID NO: 6), hsa-miR-320a (SEQ ID NO: 7), hsa-miR-320b (SEQ ID NO: 8), hsa-miR-320c (SEQ ID NO: 9).

The use may include diagnosing the progression of the disease.

The panel may include hsa-miR-483-5p (SEQ ID NO: 5) and the level of hsa-miR-483-5p (SEQ ID NO: 5) is used in assessing the progression of the disease.

The use may include differentiating whether the subject is suffering from Alzheimer's disease or Mild Cognitive Impairment due to Alzheimer's disease.

In a further aspect, the panel additionally may include at least one of microRNA(s) hsa-miR-423-5p (SEQ ID NO: 10) and/or hsa-miR-126-5p (SEQ ID NO: 11) and/or hsa-miR-22-5p (SEQ ID NO: 12) and/or hsa-miR-335-3p (SEQ ID NO: 13).

The blood sample used for assessment may have a volume of at most about 0.6 ml.

The assessment of the level of said biomarker microRNA(s) may be done by a qRT-PCR method.

The subject may be human.

Also disclosed is a kit for diagnosing Alzheimer's disease and/or Mild Cognitive Impairment (MCI) due to Alzheimer's disease and/or Subjective Cognitive Impairment (SCI) due to Alzheimer's disease, in particular for detection of an early stage of Alzheimer's disease in subjects with Mild Cognitive Impairment or with Subjective Cognitive Impairment and/or for diagnosis of Alzheimer's disease in patients with clinical dementia in a subject, wherein the kit comprises:

- instructions for use;
- means for separating plasma from blood cells to avoid hemolysis;
- means for RNA isolation and preferably RNA isolation controls;
- means for cDNA synthesis by reverse transcription, preferably with a cDNA synthesis control to evaluate the reaction; and
- a panel, preferably a qPCR panel, of primers for miRNA(s) to be used as biomarkers

wherein the miRNA(s) to be used as biomarkers include at least one of hsa-miR-151a-5p (SEQ ID NO: 1), hsa-miR-30b-5p (SEQ ID NO: 2), hsa-miR-486-5p (SEQ ID NO: 3), hsa-miR-33a-5p (SEQ ID NO: 4), hsa-miR-483-5p (SEQ ID NO: 5), hsa-miR-18a-5p (SEQ ID NO: 6), hsa-miR-320a (SEQ ID NO: 7), hsa-miR-320b (SEQ ID NO: 8), hsa-miR-320c (SEQ ID NO: 9),
preferably additionally at least one of hsa-miR-502-3p (SEQ ID NO: 14), hsa-miR-103a-3p (SEQ ID NO: 15), hsa-miR-301a-3p (SEQ ID NO: 16), hsa-miR-142-3p (SEQ ID NO: 17), hsa-miR-200a-3p (SEQ ID NO: 18), hsa-miR-1260a (SEQ ID NO: 19), and/or
preferably at least one of hsa-miR-423-5p (SEQ ID NO: 10) and/or hsa-miR-126-5p (SEQ ID NO: 11) and/or hsa-miR-22-5p (SEQ ID NO: 12) and/or hsa-miR-335-3p (SEQ ID NO: 13).

Also disclosed is a panel of biomarker microRNAs for use in a method of discriminating early versus later stage of Alzheimer's disease in a subject, characterized in that the panel comprises at least one, preferably two microRNAs selected from hsa-miR-423-5p (SEQ ID NO: 10) and/or hsa-miR-126-5p (SEQ ID NO: 11) hsa-miR-22-5p (SEQ ID NO: 12) and/or hsa-miR-335-3p (SEQ ID NO: 13);
and the method involves assessing the level of said biomarker microRNAs in subject blood sample.

The object of the invention is a method of *ex vivo* diagnosing Alzheimer's disease and/or an early stage of Alzheimer's disease, in a subject, characterized in that it involves the following steps:

- in vitro assessment of a level of at least hsa-miR-483-5p (SEQ ID NO: 5) and hsa-miR-486-5p (SEQ ID NO: 3) as biomarker microRNAs in the subject's blood sample, and

- comparison of the assessed level of biomarker microRNA(s) with the level observed for a non-demented control.

Another object of the invention is use of a panel of biomarker microRNAs comprising hsa-miR-483-5p (SEQ ID NO: 5) and hsa-miR-486-5p (SEQ ID NO: 3) for diagnosing Alzheimer's disease and/or an early stage of Alzheimer's disease in a subject by analysing the level of said biomarker microRNAs *ex vivo* in a blood sample of said subject.

Preferably, in a method or use, additionally a level of at least one additional biomarker microRNA is assessed, wherein the additional biomarker microRNA is selected from the group consisting of hsa-miR-502-3p (SEQ ID NO: 14), hsa-miR-103a-3p (SEQ ID NO: 15), hsa-miR-301a-3p (SEQ ID NO: 16), hsa-miR-142-3p (SEQ ID NO: 17), hsa-miR-200a-3p (SEQ ID NO: 18), hsa-miR-1260a (SEQ ID NO: 19), wherein preferably the assessed biomarker microRNA(s) include hsa-miR-200a-3p (SEQ ID NO: 18).

Preferably, the method or use involves additionally assessing the level of

(a) at least one, preferably two, preferably three, preferably four, preferably five, preferably six, preferably all seven, biomarker microRNAs selected form a group of hsa-miR-151a-5p (SEQ ID NO: 1), hsa-miR-30b-5p (SEQ ID NO: 2), hsa-miR-33a-5p (SEQ ID NO: 4), hsa-miR-18a-5p (SEQ ID NO: 6), hsa-miR-320a (SEQ ID NO: 7), hsa-miR-320b (SEQ ID NO: 8), hsa-miR-320c (SEQ ID NO: 9), and/or

(b) hsa-miR-502-3p (SEQ ID NO: 14), hsa-miR-103a-3p (SEQ ID NO: 15), hsa-miR-301a-3p (SEQ ID NO: 16), hsa-miR-142-3p (SEQ ID NO: 17), hsa-miR-200a-3p (SEQ ID NO: 18), and hsa-miR-1260a (SEQ ID NO: 19).

Preferably, the method includes additional assessment of a level of at least one of biomarker microRNA(s) hsa-miR-423-5p (SEQ ID NO: 10) and/or hsa-miR-126-5p (SEQ ID NO: 11) and/or hsa-miR-22-5p (SEQ ID NO: 12) and/or hsa-miR-335-3p (SEQ ID NO: 13).
Preferably, the blood sample has a volume of at most about 0.6 ml.
Preferably, the assessment of the level of said biomarker microRNA(s) is done by a qRT-PCR method.
Preferably, the subject is human.
Disclosed is also a method of discriminating early versus later stage of Alzheimer's disease in subject, which involves the following steps:

- in vitro assessment of a level of at least one of biomarker microRNAs in a subject blood sample, wherein the biomarker microRNAs are selected from a group of hsa-miR-423-5p (SEQ ID NO: 10) and/or hsa-miR-126-5p (SEQ ID NO: 11), hsa-miR-22-5p (SEQ ID 12) and/or hsa-miR-335-3p (SEQ ID 13)

- comparison of the assessed level of biomarker microRNA(s) with the level observed for a non-demented control.

The subject may be human.
Also disclosed is a use of a panel of biomarker microRNAs comprising at least one of hsa-miR-423-5p (SEQ ID NO: 10) and/or hsa-miR-126-5p (SEQ ID NO: 11) and/or hsa-miR-22-5p (SEQ ID NO: 12) and/or hsa-miR-335-3p (SEQ ID NO: 13) for discriminating early versus later stages of Alzheimer's disease in a subject by analyzing a blood sample of said subject.
The subject may be human.
The term "later Alzheimer's disease", "later stage of Alzheimer's disease" or "later AD" is intended to mean any stage of Alzheimer's disease that is more advanced then early AD in Mild Cognitive Impairment, and includes moderate and advance stages of AD.
The method can detect the early AD stage in patients with MCI, and in patients with SCI. Moreover, the test can identify AD in patients with clinical dementia.
The test overcomes the high cost and access-to-care barriers of currently available AD diagnostic assays and fulfills the optimal criteria indicated by the Food and Drug Administration (FDA) for clinical practice.

- The test is much less expensive, significantly simpler for handling, much easier to use and more immediately implementable in clinical settings, than any of the existing biochemical or brain imaging assays.
- The test can be performed in blood samples, which are easy to obtain in a safe, non-invasive clinical procedure (currently no blood-based tests approved for AD diagnosis exist).
- The test can be performed on a very small volume of blood: only as little as 0.6 ml blood is required.
- Comparing with the currently performed CSF-based tests, which require an invasive procedure of lumbar puncture and often are not feasible in elderly patients, the proposed test minimizes the risk and discomfort to patients and therefore broadens its applicability in an ageing population.
- Because of the above characteristics, the test can become the primary tool in a large-scale AD diagnosis campaign and a first step in a multistage AD diagnostic process, to identify those who should undergo further testing, involving more costly procedures and sophisticated equipment.
- The test can be useful for large-scale, cost-efficient diagnostics in clinics, lacking specialized equipment, this being a key advantage for the capabilities of diagnostic laboratories in the increasingly global threat of AD.
- The test could be useful for monitoring therapeutic interventions, especially for repeated measures.
- The test can also be used for second line screening, for the confirmation of AD diagnoses obtained based on other diagnostic tools.
- This is the first blood-based miRNA panel that can detect early AD in SCI patients.
- The test offers the possibility of diagnosing early AD in MCI and SCI patients. Easily available circulating biomarkers for early AD are desperately needed because mounting evidence indicates that the effectiveness of therapeutic modalities critically depends on the early diagnosis of AD.

- The test was based on the recommended universal blood pre-analytical protocol and thus facilitates increased reproducibility through the elimination of pre-analytical variability among diagnostic centers. The test also fosters cross-validation across cohorts and laboratories. The detailed procedure employed for blood sample collection adheres to the current guidelines for the standardization of pre-analytic variables for blood-based biomarkers [18]. The lack of success in the identification of adequate blood-based AD biomarkers is currently seen as largely the result of substantial variability among the methodologies of blood handling across various diagnostic centers [18].
- The test also offers increased probability of reproducibility because the miRNA-based blood AD signature was determined in AD and MCI subjects, in which AD diagnosis was based not only on neuropsychological testing but was additionally supported by the results of the CSF assay.
- The identified miRNAs can separate AD patients from control non-demented individuals as well as MCI patients with early AD from control non-demented individuals with adequate sensitivity and specificity.
- This is the first blood-based panel and method that can differentiate early from later AD stages. Biomarkers for the differentiation of early AD from later AD are required in order to monitor the efficacy of existing and novel therapies, and also for the proper recruitment of AD patients for clinical trials at the stage when a pharmacological intervention is still possible and could be beneficial.

Described is a method for the diagnosis of Alzheimer's disease (AD) in patients with Mild Cognitive Impairment (MCI), in patients with Subjective Cognitive Impairment (SCI), and also in patients with clinical dementia. More specifically, the inventors found 9 miRNAs which have not been reported previously in blood in any context related to AD, which show a consistently changed expression pattern in blood plasma during both early and later stages of AD, in MCI, AD and SCI patients, as compared to blood samples from non-demented age-matched subjects. Thus, one AD blood-based miRNA biomarker panel consists of: hsa-miR-151a-5p, hsa-miR-30b-5p, hsa-miR-486-5p, hsa-miR-33a-5p, hsa-miR-483-5p, hsa-miR-18a-5p, hsa-miR-320a, hsa-miR-320b, hsa-miR-320c (Tab. 1). The detection assay of miRNAs in blood plasma was based on the quantitative real time polymerase chain reaction (qRT-PCR). The reliability of the inventors' methodology for assessment of miRNA levels in blood was confirmed using 6 control miRNAs which were reported previously as linked with AD pathology (Tab. 1); these miRNAs were previously reported in 4 different studies as miRNAs whose levels were changed in AD patients: 4 miRNAs in blood of AD patients (hsa-miR-502-3p [28], hsa-miR-103a-3p [22], hsa-miR-301a-3p and hsa-miR-142-3p [21]); and 2 miRNAs in AD brain (hsa-miR-200a-3p and hsa-miR-1260a [33]. The inventors found that these 6 miRNAs were indeed dysregulated in blood samples from AD patients comparing to non-demented age-matched controls.

The novel finding in the inventors' study is also the identification of 4 miRNAs hsa-miR-423-5p, hsa-miR-126-5p, hsa-miR-22-5p and hsa-miR-335-3p in blood serum which can clearly differentiate patients in early versus later AD stage, i.e. patients with MCI due to AD (MCI-AD) from patients in more advanced (later) AD stage (AD) (Tab. 1, AD vs MCI-AD).

**Tab. 1.** The AD blood-based miRNA biomarker panel.

| miRNA | Reference database | SEQ ID NO | Sequence |
|---|---|---|---|
| New miRNA biomarkers: early and later AD (MCI-AD, AD) vs control | | | |
| hsa-miR-151a-5p | miRBase &Exiqon | 1 | UCGAGGAGCUCACAGUCUAGU |
| hsa-miR-30b-5p | miRBase &Exiqon | 2 | UGUAAACAUCCUACACUCAGCU |
| hsa-miR-486-5p | miRBase &Exiqon | 3 | UCCUGUACUGAGCUGCCCCGAG |
| hsa-miR-33a-5p | miRBase &Exiqon | 4 | GUGCAUUGUAGUUGCAUUGCA |
| hsa-miR-483-5p | miRBase &Exiqon | 5 | AAGACGGGAGGAAAGAAGGGAG |
| hsa-miR-18a-5p | miRBase &Exiqon | 6 | UAAGGUGCAUCUAGUGCAGAUAG |
| hsa-miR-320a | miRBase &Exiqon | 7 | AAAAGCUGGGUUGAGAGGGCGA |
| hsa-miR-320b | miRBase &Exiqon | 8 | AAAAGCUGGGUUGAGAGGGCAA |
| hsa-miR-320c | miRBase &Exiqon | 9 | AAAAGCUGGGUUGAGAGGGU |
| New miRNA biomarkers: early vs later AD (MCI-AD vs AD) | | | |
| hsa-miR-423-5p | miRBase &Exiqon | 10 | UGAGGGGCAGAGAGCGAGACUUU |
| hsa-miR-126-5p | miRBase &Exiqon | 11 | CAUUAUUACUUUUGGUACGCG |

(continued)

| New miRNA biomarkers: early vs later AD (MCI-AD vs AD) | | | |
|---|---|---|---|
| hsa-miR-22-5p | miRBase &Exiqon | 12 | AGUUCUUCAGUGGCAAGCUUUA |
| hsa-miR-335-3p | miRBase &Exiqon | 13 | UUUUUCAUUAUUGCUCCUGACC |
| Published miRNAs confirmed in the study: early and later AD (MCI-AD, AD) vs control | | | |
| hsa-miR-502-3p | miRBase &Exiqon | 14 | AAUGCACCUGGGCAAGGAUUCA |
| hsa-miR-103a-3p | miRBase &Exiqon | 15 | AGCAGCAUUGUACAGGGCUAUGA |
| hsa-miR-301a-3p | miRBase &Exiqon | 16 | CAGUGCAAUAGUAUUGUCAAAGC |
| hsa-miR-142-3p | miRBase &Exiqon | 17 | UGUAGUGUUUCCUACUUUAUGGA |
| hsa-miR-200a-3p | miRBase &Exiqon | 18 | UAACACUGUCUGGUAACGAUGU |
| hsa-miR-1260a | miRBase &Exiqon | 19 | AUCCCACCUCUGCCACCA |
| Control miRNAs in the verification experiment (Stage 2) | | | |
| hsa-miR-185-5p | miRBase &Exiqon | 20 | UGGAGAGAAAGGCAGUUCCUGA |
| hsa-miR-128-3p | miRBase &Exiqon | 21 | UCACAGUGAACCGGUCUCUUU |
| hsa-miR-130b-3p | miRBase &Exiqon | 22 | CAGUGCAAUGAUGAAAGGGCAU |
| hsa-miR-15a-5p | miRBase &Exiqon | 23 | UAGCAGCACAUAAUGGUUUGUG |
| hsa-miR-425-3p | miRBase &Exiqon | 24 | AUCGGGAAUGUCGUGUCCGCCC |

The present invention provides a novel method of determining whether a person is afflicted with AD even at the early stage of the disease. This method is less invasive, less expensive, faster, and more available in a clinical setting than any other approved method for supporting AD diagnostics, such as conventional CSF biochemical assays or brain imaging techniques. Moreover, the inventors have delivered the first available method to differentiate between early and later AD stages based on a biochemical assay of bodily fluids.

The test for identifying patients with early and/or later stage AD involves the following steps: obtaining the test sample through the withdrawal of merely as little as 0.6 ml of blood from a patient, obtaining a plasma/serum sample through centrifugation, isolating miRNAs, and performing an analysis of any of the 9 miRNAs identified in this study or any combination thereof using qRT-PCR.

The test to discriminate early versus later stage AD utilizes the same method of obtaining blood plasma and miRNA isolation and is also based on a qRT-PCR analysis of four additional miRNA levels: hsa-miR-423-5p and hsa-miR-126-5p, hsa-miR-22-5p, and hsa-miR-335-3p. They are differentially expressed in fully developed AD comparing to early AD in a way that allows to separate early from later AD patients with adequate specificity and sensitivity.

One of the novel aspects in the study was that all involved patients including MCI patients were diagnosed based not only on the neuropsychological tests, neurological examination, and neuroimaging, but also using canonical AD CSF biomarkers. In the previous studies of miRNA AD signatures in blood, the CSF markers assessment was mostly absent [20-22; 24-28,30] or available only for AD patients [23,29]. The diagnostic approach in the present study enabled analysis of miRNA in blood plasma not only in the later AD stages, but also in early AD stage, which is a further novel aspect of this study. This is the first study with the involvement of MCI patients in the early AD stage, i.e. MCI with a high likelihood that the MCI was due to AD as confirmed by the levels of CSF markers (patients denoted as MCI-AD). The inventors compared early AD patients (MCI-AD) as well as later AD patients (AD) with two separate, non-demented, age-matched control groups. The panel of miRNAs biomarkers identified in AD and in MCI-AD patients was further verified in the MCI patients with low indications for AD (group denoted MCI) and in subjective cognitive impairment (SCI) patients with two slightly positive CSF AD biomarkers.

Obtained results indicated a panel of 9 novel miRNAs in blood which clearly separated AD as well as MCI-AD patients from non-demented control subjects. Furthermore, the panel of the 9 novel blood miRNAs distinguished MCI patients with low indication for AD from non-demented control subjects. Furthermore, the panel of the 9 novel blood miRNAs distinguished also patients with subjective cognitive impairment (SCI) with two slightly positive AD CSF markers from non-demented control subjects. Thus the results suggest that the miRNAs blood panel can replace CSF markers for identification of early and later AD. Moreover, it can detect early AD in SCI patients with slight indication for AD and in MCI patients with low indication for AD according to levels of the CSF biomarker.

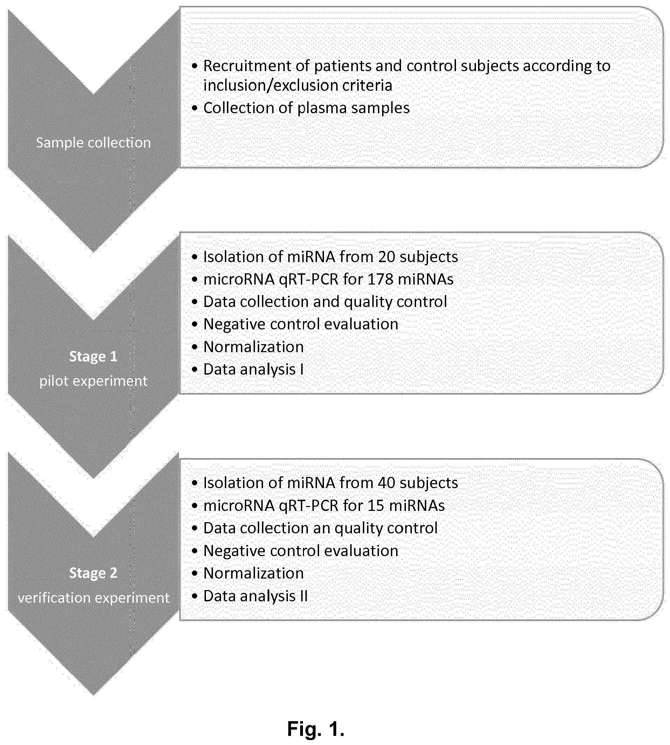

The study design is schematically shown in Fig. 1. The study consisted of two main stages of profiling miRNA in blood plasma samples: the pilot experiment (Stage 1, train set) and the verification experiment (Stage 2, test set). In the first stage (pilot experiment), the inventors used qRT-PCR-based Exicon microarrays for assessment of blood plasma levels of 178 miRNAs. These 178 miRNAs were found previously in total human blood plasma, and were selected based on the data collected in the Exicon database. The pilot experiment was performed in three groups of individuals: in healthy non-demented subjects aged-matched to patients (control group 1, CTR1), in patients diagnosed with AD (AD1), and in patients with early AD, i.e. diagnosed with MCI with high likelihood that MCI was due to AD (MCI-AD1), (Tab. 2.). This pilot experiment led to the identification of 15 miRNAs expressed differentially, with the largest fold changes differentiating both AD1 versus control CTR1, and MCI-AD1 versus control CTR1 (control group 1), with statistical significance evaluated using a t-test and ANOVA. Among these 15 miRNAs, 9 miRNAs were not previously reported in the context of AD, and particularly were not reported as the potential AD blood biomarkers. All the 15 miRNAs have been chosen for validation in the second stage of the study, in separate, subsequent groups of subjects.

Furthermore, during the comparison of the miRNA profile of the MCI-AD1 group in early AD stage to the AD1 group in later AD stage, the inventors have found that four miRNAs were differentially expressed with the highest fold changes between early and later AD patients. Thus these four miRNAs can also be used in the method allowing for additional differentiation and analysis of the progress of the disease.

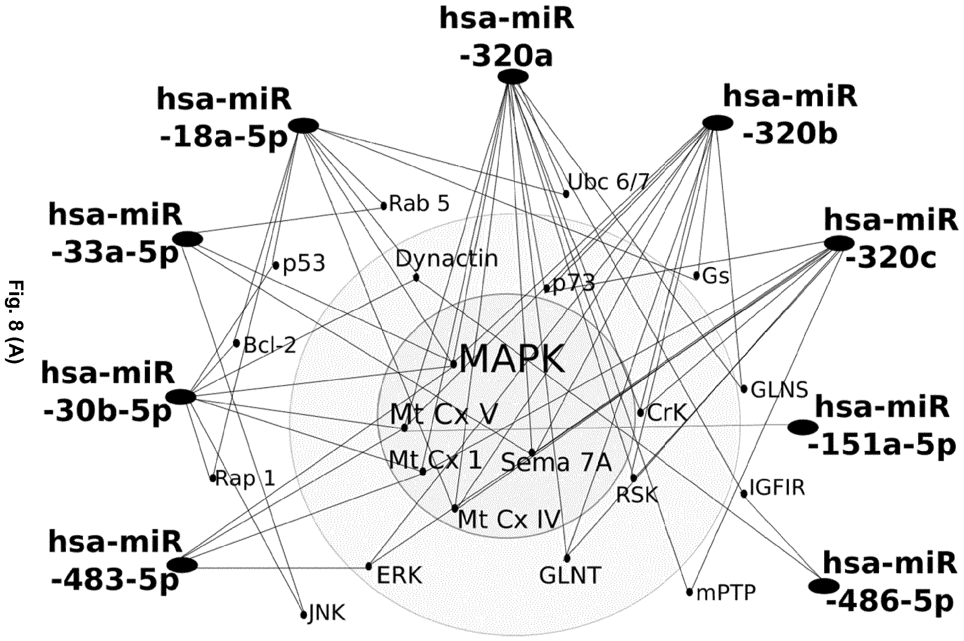

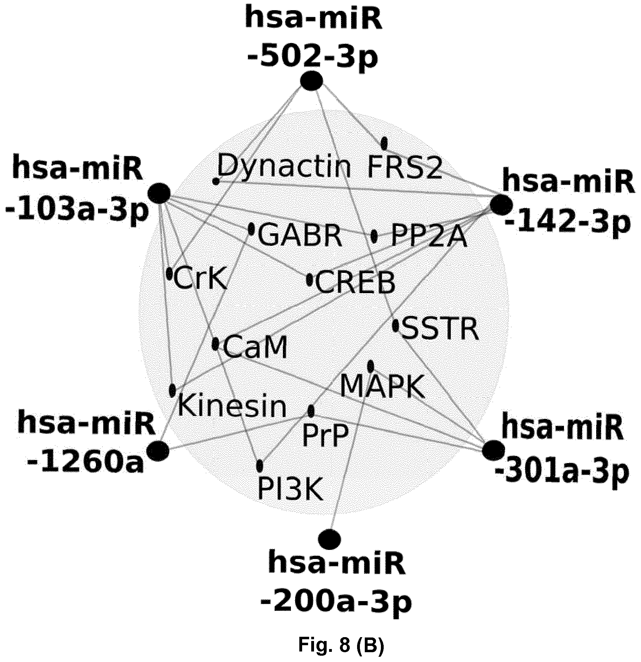

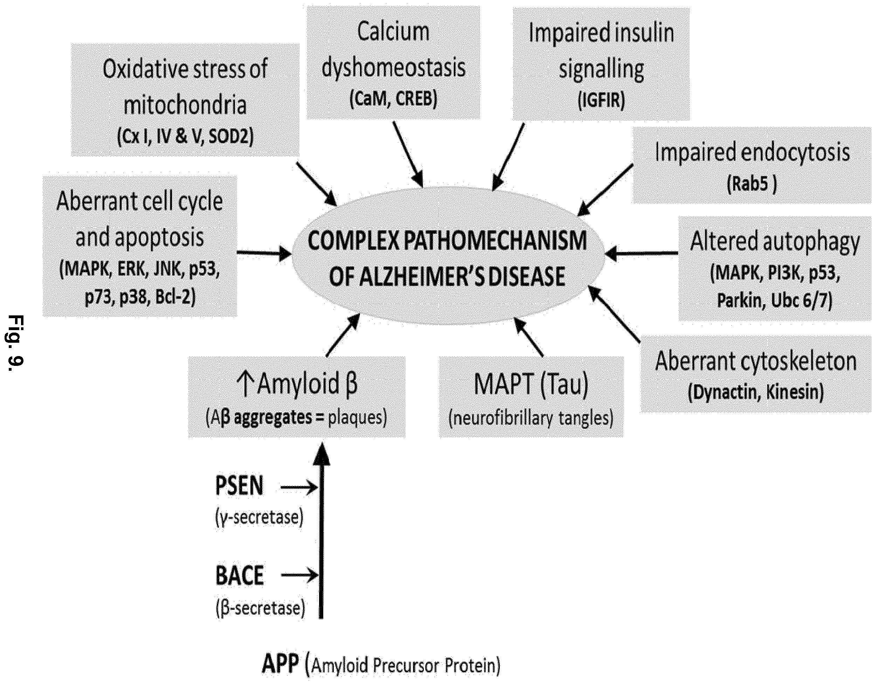

Furthermore, the miRNAs identified by the inventors herein were linked to a group of downstream target genes/proteins known to contribute to AD pathogenesis. The network of targets centered around the key proteins for AD pathogenesis including MAPT (tau) protein, Amyloid Precursor Protein (APP) and enzymes involved in the production of the toxic amyloid. Moreover, the targets included proteins of the mitochondria respiratory chain (Cxi, CxIV, CxV) that were implicated in oxidative stress in AD pathology, and several other proteins known to take center stage in aberrant cellular signaling in AD pathology such as MAPK, insulin growth factor receptor I (IGFR I), apoptosis-related proteins p53 and Bcl-2, and proteins involved in autophagy, endocytosis, cytoskeleton regulation and calcium signaling (Tab. 8, 9 and Fig. 8, 9). It is known that AD is a complex, heterogeneous disease with several impaired signaling pathways contributing to its pathomechanism, as shown schematically in Fig. 9. Due to AD heterogeneity, in addition to some common AD features as amyloidosis and tauopathy, individual patients show disease endophenotypes differing in the composition of signaling pathways/proteins contributing to the disease. Thus, the identified miRNAs are further validated and corroborated as being particularly relevant and suitable for the diagnostic purposes described herein, as they can be related to the molecular targets that are relevant to the individual AD endophenotype of a patient. Therefore the analysis of the miRNAs and groups and panels thereof as disclosed herein serve not only as basic molecular signatures relating to AD, but also as a reflection of complex AD pathology. Thus, for example, the miRNAs, groups and panels thereof as disclosed herein allow patients to be sub-classified for further targeted and/or personalized therapy and for treatment effectiveness to be monitored.

**BRIEF DESCRIPTION OF DRAWINGS**

**Fig. 1** shows the study design scheme.

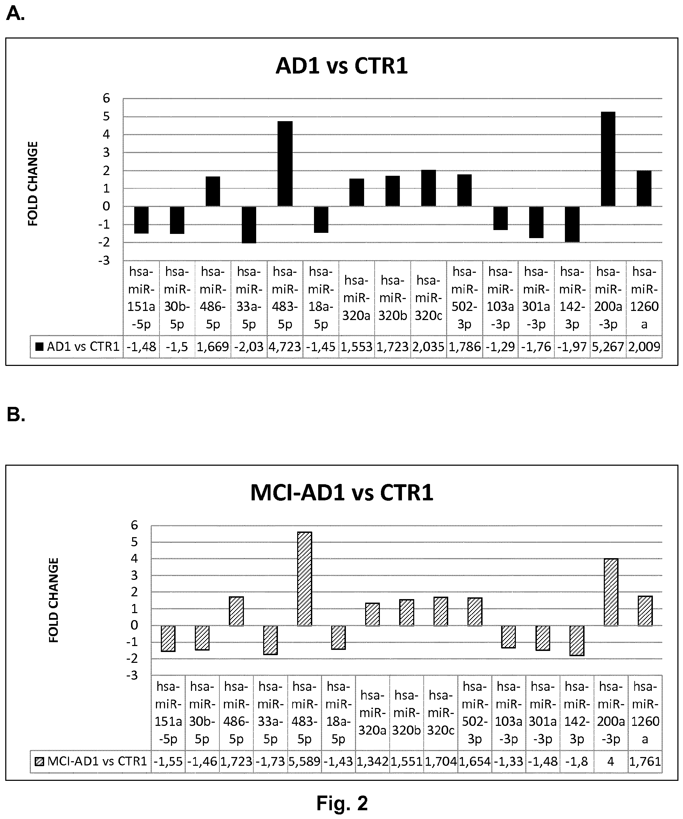

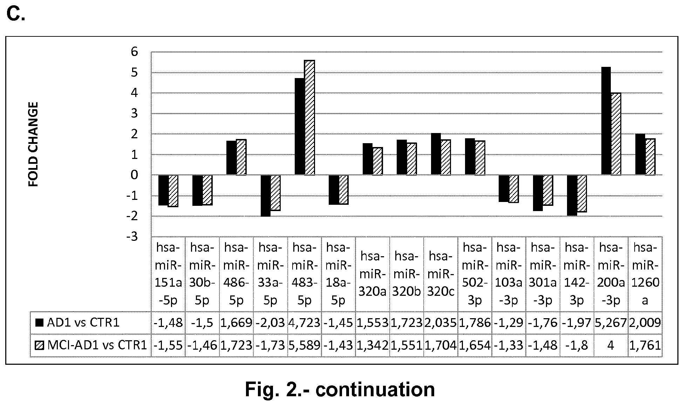

**Fig. 2** shows the fold changes in the expression levels determined by qRT-PCR in the Stage 1 of the study for the 15-miRNAs with the highest fold changes between: (A) later AD patients AD1 versus non-demented controls CTR1, and (B) early AD patients MCI-AD1 versus non-demented controls CTR1. Fig. 2C shows the data for later and early AD patients versus non-demented control on one plot (AD1 and MCI-AD1 vs CTR1).

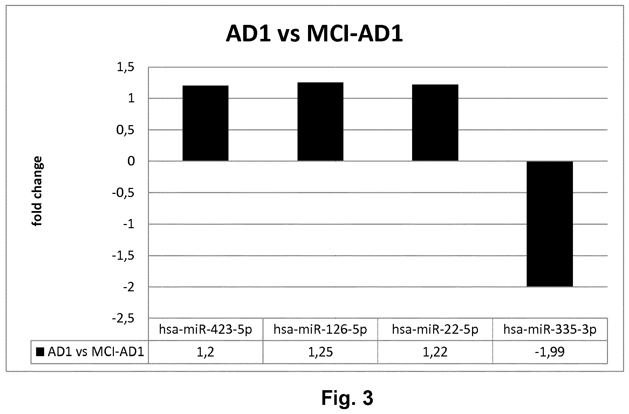

**Fig. 3** shows the fold changes in the expression levels obtained by qRT-PCR for the four miRNAs: hsa-miR-423-5p, hsa-miR-126-5p, hsa-miR-22-5p, and hsa-miR-335-3p differentiating samples from later AD versus early AD (AD1 patients versus MCI-AD1 patients).

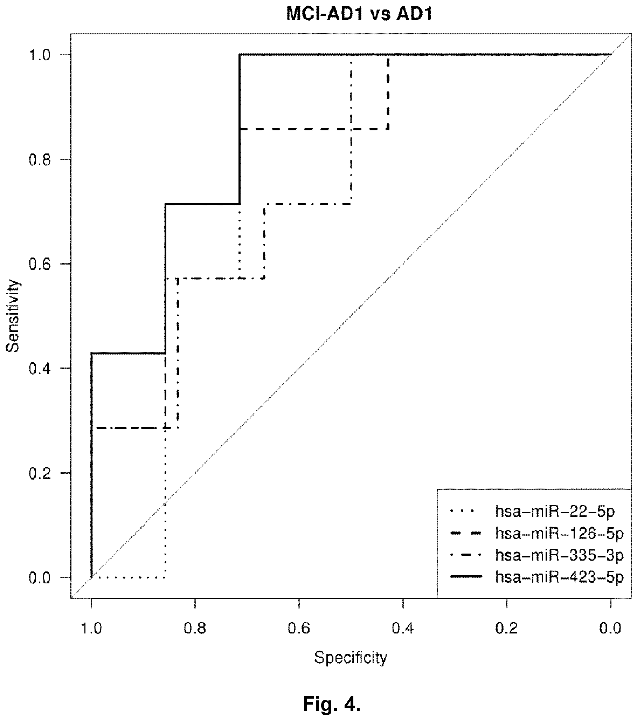

**Fig. 4.** shows that the four miRNAs: hsa-miR-423-5p, hsa-miR-126-5p, hsa-miR-22-5p, and hsa-miR-335-3p clearly distinguish early from later AD as shown on the receiver operating characteristic (ROC) curves. ROC curves were prepared based on the miRNAs expression levels in patients with early AD (MCI-AD1 group) versus patients in later AD stages (AD1 group).

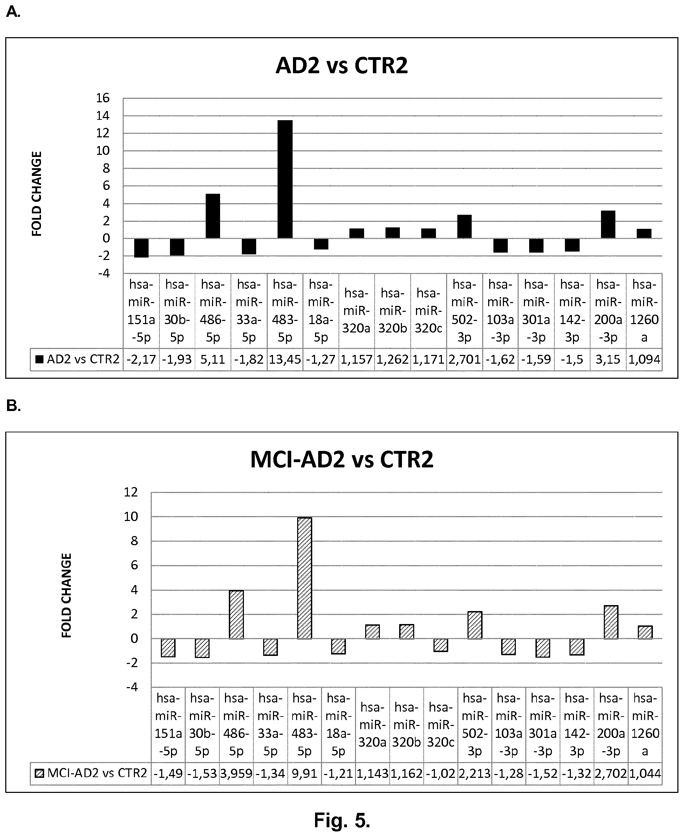

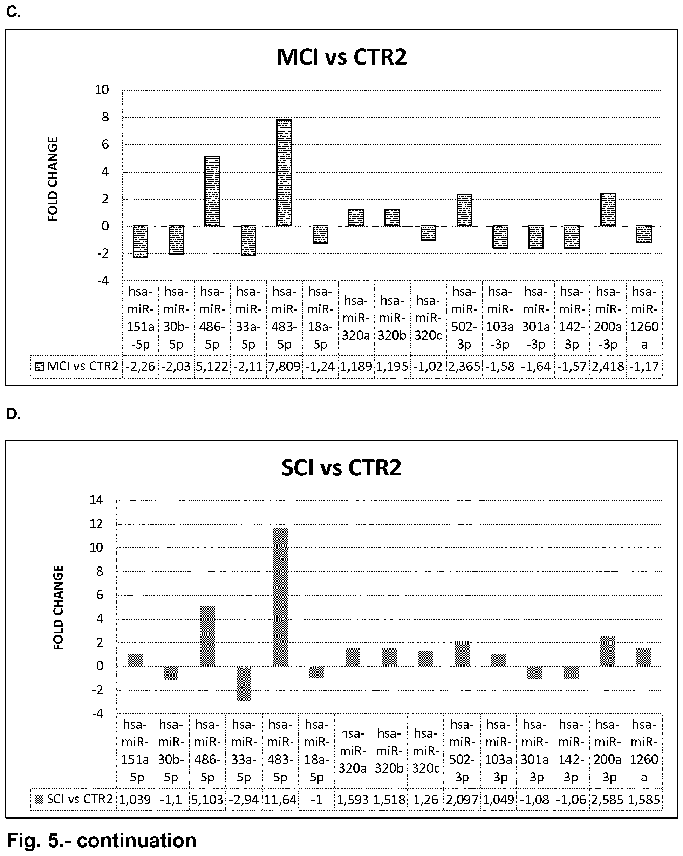

**Fig. 5.** shows the fold changes in the expression levels obtained by qRT-PCR in Stage 2 of the study for the 15-miRNA signatures differentiating patient groups from the non-demented control group CTR2. (A) AD patients AD2 versus controls CTR2, (B) early AD patients MCI-AD2 versus controls CTR2, (C) MCI patients with low indication for AD versus controls CTR2, (D) SCI patients with slight indication for AD versus controls CTR2.

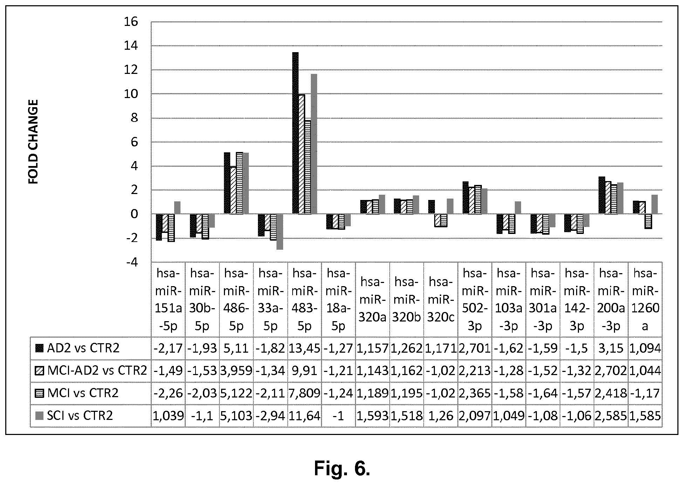

**Fig. 6.** shows the comparison of the fold changes in the expression levels obtained by qRT-PCR for the 15-miRNA signatures which differentiated AD2, MCI-AD2, MCI, and SCI patients versus non-demented controls CTR2.

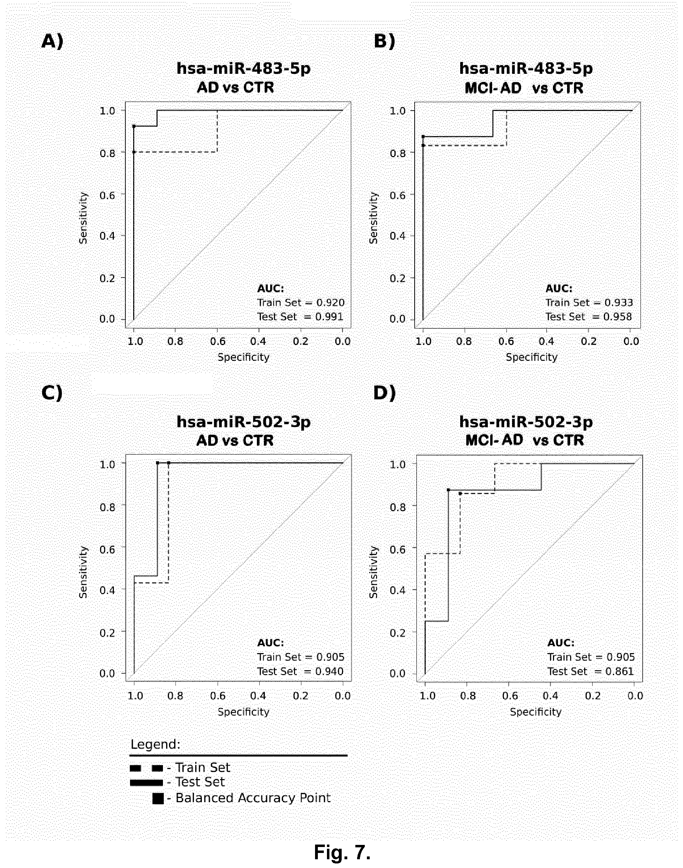

**Fig. 7.** shows that the two examled miRNAs: hsa-miR-483-5p (SEQ ID NO: 5) and hsa-miR-502-3p (SEQ ID NO: 14) clearly distinguish with a high sensitivity and specificity both early AD as well as later AD patients from non-demented controls. The receiver operating characteristic (ROC) curves were prepared for two sets of samples called a train set (dashed line; data of the Stage 1, pilot experiment), and test set (solid line; data from the Stage 2, verification experiment) to confirm the ability of each miRNA to properly separate the samples. ROC curve parameters for all analyzed 15 miRNAs are shown in Tab. 7. AD, samples from all patients with AD; MCI-AD, samples from all patients with MCI due to AD (in early AD stage); CTR, control samples from all non-demented individuals. Fig. 7A, C, comparison of control samples against AD samples (A - hsa-miR-483-5p, SEQ ID NO: 5; C - hsa-miR-502-3p, SEQ ID NO: 14); Fig. 7B, D, comparison of control samples against MCI-AD samples (B - hsa-miR-483-5p, SEQ ID NO: 5; D - hsa-miR-502-3p, SEQ ID NO: 14).

**Fig. 8. shows schematically the regulatory network of 15 AD biomarker miRNAs and their cellular effectors.** Putative targets were identified by searching the MirTarBase (version 6) database which contains experimentally validated miRNA target genes, followed by a KEGG's database search for pathways contributing to neurodegenerative diseases and pathways of the nervous system. In A, the inner circle contains targets common for at least 4 and up to 6 miRNAs, the middle layer contains targets common for 3 miRNAs, and the outer layer contains targets for 2 miRNAs. In B, targets shown are for 2 miRNAs. Highlighted are the miRNAs with a higher number of hits to putative protein targets.

**Fig. 9. shows schematically the contribution of different proteins and cellular signaling pathways to the complex pathomechanism of Alzheimer's disease.** The proteins identified as targets of blood-based 15 biomarker miRNAs are shown.

EXAMPLES

**Example 1: pilot screening for miRNA biomarkers**

The pilot experiment was performed in the following three groups of subjects (Tab.2): in 7 patients diagnosed with clinical AD (AD1), in 7 patients diagnosed with MCI due to AD (early AD, MCI-AD1) (Tab. 11), and in 6 healthy non-demented age-matched individuals (CTR1).

**Tab. 2.** Characteristics of subject groups enrolled in the Stage 1 experiments. Detailed patient diagnostic data are shown in Tab. 11

| Sample group | Cohort size | Age (mean+/- SD) | Sex (female/male) |
|---|---|---|---|
| **AD1** | 7 | 72 +/-5 | 3/4 |
| **MCI-AD1** (early AD) | 7 | 62+/-6 | 3/4 |
| **CTR1** (non-demented, healthy subjects) | 6 | 66+/-5 | 4/2 |

In the first stage (pilot experiment), the inventors used qRT-PCR-based Exiqon microarrays which allow the assessment of 178 miRNA levels (miRCURY LNA focus panel). These 178 miRNAs in the focus panel were carefully selected based on over 1 million data points from human blood serum/plasma samples collected from healthy as well as diseased individuals available at Exiqon and at collaborative sources. For the selection of the relevant miRNAs for this panel, miRNA expression data from various types of cancer, neurological disorders/neurodegenerative diseases and allergies/inflammation were used. Importantly, Exiqon's database is largely based on total miRNA extracted from human blood plasma or serum, meaning that a particular circulating miRNA can be detected by the applied focus panel irrespective of whether it is preferentially protein bound, found in exosomes, micro vesicles or otherwise compartmentalized, resulting in a panel allowing for a very comprehensive blood miRNA assay.

All samples of miRNA isolated from each subject were analyzed by standardized Exiqon's methodology on miRCURY LNA panel by the qRT-PCR using Roche Lightcycler 480.

The analysis of the pilot study results showed that an average of 170 miRNAs out of 178 were detected per sample. The minimal number of identified miRNAs in a sample was 153. For the normalization of the data, the inventors applied the average of the assays detected in all samples (n=20 samples), and this was the stable normalizer. The inventors performed a pairwise test between all three groups and an ANOVA test. The analysis identified differences between the analyzed groups. When comparing the groups in 'Class' using a one-way ANOVA, 32 miRNAs were found to be differentially expressed using a p-value cutoff of < 0.05.

The miRNA profile comparison between the AD1 group and the control group (CTR1) indicated 30 differentially expressed miRNAs using a t-test cutoff of p-value < 0.05 (Tab. 3.). In turn, when comparing the miRNA profile of MCI-AD1 group (early AD) to the control group, 38 miRNAs were found to be differentially expressed using a cutoff of p-value < 0.05 (Tab. 3).

**Tab. 3.** miRNAs found using a t-test p-value cutoff of < 0.05 to be differentially expressed among the following groups: AD patients (AD1), MCI due to AD patients (MCI-AD1), healthy non-demented age-matched controls (control group 1, CTR1) .

| Comparison Differentially expressed miRNAs | |
|---|---|
| **AD1 vs CTR1** | hsa-let-7d-3p, hsa-miR-423-5p, hsa-miR-30b-5p, hsa-miR-200a-3p, hsa-miR-142-3p, hsa-let-7g-5p, hsa-miR-320c, hsa-miR-320a, hsa-miR-2110, hsa-miR-320b, hsa-miR-15b-3p, hsa-let-7f-5p, hsa-let-7b-5p, hsa-miR-18a-5p, hsa-miR-10b-5p, hsa-miR-301a-3p, hsa-miR-1260a, hsa-miR-502-3p, hsa-miR-374a-5p, hsa-miR-483-5p, hsa-let-7d-5p, hsa-miR-103a-3p, hsa-miR-574-3p, hsa-miR-33a-5p, hsa-miR-320d, hsa-miR-22-5p, hsa-miR-374b-5p, hsa-miR-193a-5p, hsa-miR-151a-5p, hsa-miR-486-5p |
| **MCI-AD1 vs CTR1** | hsa-miR-142-3p, hsa-miR-33a-5p, hsa-miR-320b, hsa-miR-502-3p, hsa-let-7b-5p, hsa-miR-30b-5p, hsa-miR-181a-5p, hsa-miR-342-3p, hsa-miR-18a-5p, hsa-miR-301a-3p, hsa-miR-151a-5p, hsa-miR-483-5p, hsa-let-7d-3p, hsa-miR-27a-3p, hsa-miR-320c, hsa-miR-146a-5p, hsa-miR-320a, hsa-miR-200a-3p, hsa-miR-103a-3p, hsa-miR-660-5p, hsa-miR-10b-5p, hsa-miR-486-5p, hsa-miR-92a-3p, hsa-miR-205-5p, hsa-miR-126-3p, hsa-let-7f-5p, hsa-miR-222-3p, hsa-miR-339-5p, hsa-miR-132-3p, hsa-miR-18b-5p, hsa-let-7g-5p, hsa-miR-374a-5p, hsa-miR-454-3p, hsa-miR-1260a, hsa-miR-150-5p, hsa-miR-27b-3p, hsa-miR-339-3p, hsa-miR-374b-5p |

The inventors finalized this analysis by the identification of 15 miRNAs differentially expressed and with the biggest fold changes between AD1 versus CTR1, and MCI-AD1 versus CTR1 groups, with statistical significance both in t-test and ANOVA analysis (Fig. 2). Importantly, all these 15 miRNAs showed the same pattern of changes, i.e. direction of change (down or up regulation) and fold change in AD1 and MCI-AD1 samples compared with CTR1. Among these 15 selected miRNAs, 9 miRNAs were newly identified, not reported previously as differentiating the blood of AD or MCI patients with respect to non-demented controls: hsa-miR-151a-5p (SEQ ID NO: 1), hsa-miR-30b-5p (SEQ ID NO: 2), hsa-miR-486-5p (SEQ ID NO: 3), hsa-miR-33a-5p (SEQ ID NO: 4), hsa-miR-483-5p (SEQ ID NO: 5), hsa-miR-18a-5p (SEQ ID NO: 6), hsa-miR-320a (SEQ ID NO: 7), hsa-miR-320b (SEQ ID NO: 8), hsa-miR-320c (SEQ ID NO: 9). In addition, among the selected 15 miRNAs, were 6 miRNAs which were previously reported in 4 different studies as miRNAs whose levels were changed in AD patients compared to controls: 4 miRNAs in blood of AD patients: hsa-miR-502-3p (SEQ ID NO: 14) [28], hsa-miR-103a-3p (SEQ ID NO: 15) [22], hsa-miR-301a-3p (SEQ ID NO: 16) and hsa-miR-142-3p (SEQ ID NO: 17) [21]; and 2 miRNAs in AD brain: hsa-miR-200a-3p (SEQ ID NO: 18) and hsa-miR-1260a (SEQ ID NO: 19) [33].

These 15 miRNAs were therefore chosen for validation in the next step of our study.

Moreover, the comparison of the miRNA profile of the MCI-AD1 group in early AD stage to the AD1 group in later AD stage indicated several miRNAs that were differentially expressed. Fig. 3 shows the 4 miRNAs which were differentially expressed with the highest fold changes between early and later AD patients: hsa-miR-423-5p (SEQ ID NO: 10) hsa-miR-126-5p (SEQ ID NO: 11), and hsa-miR-22-5p (SEQ ID NO: 12) were upregulated while hsa-miR-335-3p (SEQ ID NO: 13) was down-regulated in more advanced (later) AD stages. Out of them two miRNAs were differentially expressed with statistical significance using a cutoff of p-value < 0.05 (hsa-miR-423-5p (SEQ ID NO: 10), hsa-miR-126-5p (SEQ ID NO: 11), Tab. 4). The miRNAs differentially expressed between early and later AD patients most probably and without wishing to be bound by theory reflect the progression of AD pathogenesis and its complexity. Receiver Operating Characteristics (ROC) curves (Fig. 4) and ROC curve parameters shown in Tab. 4. document that these four differentially expressed miRNAs clearly separate patients with early AD from patients with more advanced (later) AD with a good accuracy.

**Tab. 4.** Receiver Operating Characteristic (ROC) curve parameters, fold change and statistical significance (P values) for the four miRNAs that separate early versus later stage AD patients (MCI-AD1 vs AD1). Parameters were calculated based on ROC curves shown in Fig. 4. AUC, area under the ROC curve.

| miRNA | SEQ ID NO | AUC | Specificity | Sensitivity | Fold change | P value |
|---|---|---|---|---|---|---|
| hsa-miR-423-5P | 10 | 0.79 | 0.67 | 0.91 | ↑ 1.20 | 0.011 |
| hsa-miR-126-5P | 11 | 0.74 | 0.62 | 0.85 | ↑ 1.25 | 0.044 |
| hsa-miR-22-5p | 12 | 0.79 | 0.65 | 0.94 | ↑ 1.22 | 0.073 |
| hsa-miR-335-3p | 13 | 0.72 | 0.54 | 0.80 | ↓ 1.99 | 0.083 |

**Example 2: Verification experiment of miRNA biomarkers in the next groups of subjects**

In the second experimental stage, the inventors used the same methodology for validating the pilot experiment results through the analysis of the levels of the 15 differential miRNAs selected in the first stage: hsa-miR-151a-5p (SEQ ID NO: 1), hsa-miR-30b-5p (SEQ ID NO: 2), hsa-miR-486-5p (SEQ ID NO: 3), hsa-miR-33a-5p (SEQ ID NO: 4), hsa-miR-483-5p (SEQ ID NO: 5), hsa-miR-18a-5p (SEQ ID NO: 6), miR-320a (SEQ ID NO: 7), hsa-miR-320b (SEQ ID NO: 8), hsa-miR-320c (SEQ ID NO: 9), hsa-miR-502-3p (SEQ ID NO: 14), hsa-miR-103a-3p (SEQ ID NO: 15), hsa-miR-301a-3p (SEQ ID NO: 16), hsa-miR-142-3p (SEQ ID NO: 17), hsa-miR-200a-3p (SEQ ID NO: 18), hsa-miR-1260a (SEQ ID NO: 19). For an additional control, 5 most stable miRNAs were assayed (hsa-miR-185-5p (SEQ ID NO: 20), hsa-miR-128-3p (SEQ ID NO: 21), hsa-miR-130b-3p (SEQ ID NO: 22), hsa-miR-15a-5p (SEQ ID NO: 23), hsa-miR-425-3p (SEQ ID NO: 24). The levels of these 20 miRNAs have been analyzed in three novel, separate groups of subjects (Tab. 5.).

**Tab. 5.** Characteristics of subject groups enrolled in the Stage 2 experiments. Detailed patient diagnostic data are shown in Tab. 10 and Tab. 11

| Sample group | Cohort size | Age (mean+/- SD) | Sex (female/male) |
|---|---|---|---|
| **AD2** | 13 | 66 +/- 7 | 7/6 |
| **MCI-AD2** (early AD) | 8 | 62 +/- 6 | 5/3 |
| **MCI** MCI patients with low indications for AD | 10 | 62 +/- 5 | 4/6 |
| **SCI** SCI patients with slight indication for AD | 3 | 61 +/- 4 | 3/0 |
| **CTR2** non-demented subjects with hypertension | 9 | 66 +/- 3 | 4/5 |

As a control group 2 (CTR2), in this stage the inventors employed samples from non-demented age-matched patients with hypertension. The inclusion/exclusion criteria are given in Tab. 12. As hypertension is one of the risk factors for AD, and often represents one of the comorbidities in AD patients, such a control group could lead to exclusion of miRNA common for both diseases and to narrow the miRNA panel to more AD-specific. The inventors analyzed 15 miRNA profiles in the second group of AD patients (AD2) in which AD diagnoses were confirmed by CSF markers and in two next groups of MCI patients: one group diagnosed with early AD based on the levels of the CSF markers (MCI-AD2) and a second group diagnosed with MCI with low indication for AD, in which CSF AD biomarkers levels were not clearly changed (MCI) (Tab. 5, Tab. 10, Tab. 11). In addition, the inventors also included a group of patients with subjective cognitive impairment (SCI-CSF), with slight indication for AD according to two slightly positive CSF markers (Tab. 5, Tab. 10).

In this example, the sample preparation and methodology of miRNA analysis using the focus panel for the qPCR-RT assay of the 15 miRNAs were identical to the ones used in the preliminary experiments, described in Example 1. For the normalization of the data, as in Example 1, the inventors applied the average of the assays detected in all samples as this was found to be the most stable normalizer. In a one-way ANOVA analysis, the inventors obtained a clear separation of the control group from AD and MCI-AD, similarly to the preliminary experiment in Stage 1. In addition, the inventors obtained separation of the control group from MCI and SCI patients with low/weak evidence for AD dementia.

This independent experiment of Example 2 confirmed the dysregulation of all of the 15 selected miRNAs in blood plasma, including 9 novel miRNA biomarkers identified in Example 1 of this study (Fig. 5).

When the inventors compared the joint results of the verification experiment, data for all 15 miRNA proved to be very consistent (Fig. 6). Significantly, the newly reported 9 miRNAs were verified as a novel AD biomarker panel which can be used as a whole panel in the AD diagnostic test. What is more, any of the 9 individual miRNAs and/or any combination of the miRNA panel markers can be employed for such a diagnostic AD test. Moreover, the panel may include any one of up to all of the 15 miRNA confirmed to be valuable in AD diagnostics.

What is important, the results of the verification experiment were consistent with the preliminary experiment. It should be stressed that in all our test groups (AD, MCI-AD, MCI, SCI) comparing to two different control groups (CTR1 and CTR2), data for the 9 miRNAs, or more preferably all 15 miRNA of the diagnostic panel, are consistent in terms of the direction of changes in miRNA levels (upregulation and downregulation) and in fold changes (Tab. 6, Fig. 6, Fig. 2C). Two of the 9 novel miRNAs showed the highest fold changes (in the range from 4 - 13), and the highest statistical significance: hsa-miR-486-5p (SEQ ID NO: 3) and hsa-miR-483-5p (SEQ ID NO: 5). In particular, in the verification experiment which was performed in larger subject groups, the fold change in case of hsa-miR-483-5p (SEQ ID NO: 5) was extremely high, in the range 8-13 (Fig. 6). In the AD literature published so far, no miRNAs were reported with fold changes above 6, when comparing AD patients to controls, and only very few miRNAs were reported with fold changes in the range 3-6 (5 miRNAs, i.e. 6.3% of all reported miRNAs). Thus, these 2 miRNAs identified in our study: hsa-miR-486-5p (SEQ ID NO: 3) and particularly hsa-miR-483-5p (SEQ ID NO: 5) are the most significant indicators of AD. Moreover, the fold change of hsa-miR-483-5p (SEQ ID NO: 5) proved to correspond well with the CSF biomarker results and disease progression: the highest fold change was detected in the AD2 group, a lower fold change in early AD in MCI-AD2 and in SCI groups with slightly positive two CSF markers, and the least in the MCI group with low indication for AD according to CSF markers (Fig. 6). In addition, a very consistent and highly statistically significant upregulation of hsa-miR-502-3p (SEQ ID NO: 14) has been observed in both Stage 1 and Stage 2 of the study, with approximately two-fold increased levels of this miRNA in AD and in MCI-AD samples compared to non-demented controls (Fig. 6, Fig. 2, Tab. 6).

Importantly, six control miRNAs: hsa-miR-502-3p (SEQ ID NO: 14), hsa-miR-103a-3p (SEQ ID NO: 15), hsa-miR-301a-3p (SEQ ID NO: 16), hsa-miR-142-3p (SEQ ID NO: 17), hsa-miR-200a-3p (SEQ ID NO: 18) and hsa-miR-1260a (SEQ ID NO: 19) were demonstrated as well differentiating control samples from AD and MCI-AD samples both in our primary and verification experiments. These miRNAs have not been accepted yet as biomarkers that enable diagnosis of early or late AD from blood.

**Tab. 6.** Directions of changes ($\downarrow$ decrease, $\uparrow$ increase) in the level of 15 differential miRNAs detected in the Example 1 (preliminary experiment) and confirmed in Example 2 (verification experiment). For 6 miRNAs which were reported in previous studies, direction of changes were compared also with the published results (references included). Data for 9 miRNAs reported here for the first time as AD biomarkers have no references (n/a). Statistical significance: (p-value): * $p \leq 0.05$, ** $p \leq 0.01$, *** $p \leq 0.001$; **** $P \leq 0.0001$. Abbreviations: pref. cortex, prefrontal cortex, hippocamp., hippocampus.

| A) Comparison of AD patient groups versus control groups | | | | | | |
|---|---|---|---|---|---|---|
| **miRNA hsa-miR** | **SEQID NO** | Pilot **AD1vsCTR1** | Verification **AD2vsCTR2** | Previously published | **Tissue** | **Ref.** |
| **151a-5p** | 1 | $\downarrow$* | $\downarrow$ **** | n/a | | |
| **30b-5p** | 2 | $\downarrow$** | $\downarrow$ ** | n/a | | |
| **486-5p** | 3 | $\uparrow$* | $\uparrow$ *** | n/a | | |
| **33a-5p** | 4 | $\downarrow$* | $\downarrow$ * | n/a | | |
| **483-5p** | 5 | $\uparrow$* | $\uparrow$ **** | n/a | | |
| **18a-5p** | 6 | $\downarrow$* | $\downarrow$ * | n/a | | |

(continued)

| A) Comparison of AD patient groups versus control groups | | | | | | |
|---|---|---|---|---|---|---|
| miRNA hsa-miR | SEQID NO | Pilot AD1vsCTR1 | Verification **AD2vsCTR2** | Previously published | **Tissue** | **Ref.** |
| **320a** | 7 | ↑ ** | ↑ | n/a | | |
| **320b** | 8 | ↑ ** | ↑ * | n/a | | |
| **320c** | 9 | ↑ ** | ↑ | n/a | | |
| **502-3p** | 14 | ↑ * | ↑ **** | n/a | Blood | [28] |
| **103a-3p** | 15 | ↓ * | ↓ ** | ↓ | Blood | [22] |
| **301a-3p** | 16 | ↓ * | ↓ ** | ↓ | Blood | [21] |
| **142-3p** | 17 | ↓ ** | ↓ * | ↓ | Blood | [21] |
| **200a-3p** | 18 | ↑ ** | ↑ *** | ↑ | Hippocamp. | [33] |
| **1260a** | 19 | ↑ * | ↑ | ↑ | Pref. cortex | [33] |

| B) Comparison for MCI patient groups versus control groups | | | | | | | |
|---|---|---|---|---|---|---|---|
| miRNA hsa-miR | SEQID NO | Pilot **MCI-AD1 vs CTR1** | Verification **MCI-AD2 vs CTR2** | **MCI vs CTR2** | Previously published | **Tissue** | **Ref.** |
| **151a-5p** | 1 | ↓ | ↓ * | ↓ ** | n/a | | |
| **30b-5p** | 2 | ↓ ** | ↓ * | ↓ ** | n/a | | |
| **486-5p** | 3 | ↑ * | ↑ ** | ↑ *** | n/a | | |
| **33a-5p** | 4 | ↓ **** | ↑ | ↑ * | n/a | | |
| **483-5p** | 5 | ↑ * | ↑ *** | ↑ *** | n/a | | |
| **18a-5p** | 6 | ↓ ** | ↓ | ↓ * | n/a | | |
| **320a** | 7 | ↑ * | ↑ | ↑ | n/a | | |
| **320b** | 8 | ↑ ** | ↑ | ↑ | n/a | | |
| **320c** | 9 | ↑ * | ↓ | ↓ | n/a | | |
| **502-3p** | 14 | ↑ ** | ↑ ** | ↑ *** | n/a | Blood | [28] |
| **103a-3p** | 15 | ↓ * | ↓ | ↓ ** | ↓ | Blood | [22] |
| **301a-3p** | 16 | ↓ ** | ↓ | ↓ ** | ↓ | Blood | [21] |
| **142-3p** | 17 | ↓ **** | ↓ | ↓ * | ↓ | Blood | [21] |
| **200a-3p** | 18 | ↑ * | ↑ * | ↑ *** | ↑ | Hippocamp. | [33] |
| **1260a** | 19 | ↑ * | ↑ | ↓ | ↑ | Pref. cortex | [33] |

To further test the suitability of each of the 15 miRNAs in the panel as biomarkers for AD, the inventors calculated the Receiver Operating Characteristics (ROC) curves. This analysis demonstrated that the 15 miRNAs clearly separate AD and MCI-AD samples from control samples with specificity, sensitivity and accuracy (area under the ROC, AUC) values given for each miRNA in Tab. 7. Furthermore, Fig. 7 shows ROC curves of two selected exampled miRNAs: of hsa-miR-483-5p (SEQ ID NO: 5) for which the fold increase was extremely high when comparing AD and MCI-AD patients to non-demented controls (8-13 fold change), and of hsa-miR-502-3p (SEQ ID NO: 14) which represents miRNAs of lower fold increase in AD and MCI-AD patients comparing to controls (approx. 2 fold change). Tab. 7 and Fig. 7 indicate that these two miRNAs separated AD patients from controls as well as MCI-AD patients (early AD) from controls with high accuracy (AUC over 0.9), repeatedly in both preliminary (train) and verification (test) studies. Thus, based on consistently high ROC curve parameters, the miRNAs hsa-miR-483-5p (SEQ ID NO: 5) and hsa-miR-502-3p (SEQ ID NO: 14) represent especially promising biomarkers.

ROC curves and AUC are considered an objective method for evaluating binary classifiers. ROC curves illustrate a classifier's performance over the range of thresholds for sensitivity and specificity. Sensitivity is the portion of correctly classified positive observations and the specificity is the portion of correctly classified negative observations. The AUC is the summary measure of accuracy which incorporate sensitivity and specificity into a single measure and quantifies the ranking ability of a ranking value (i.e. here expression level of single miRNA). It ranges from 0 to 1. A higher AUC means better classification. A perfect classifier will have ROC curve passing through (1,1) and AUC of 1 (upper left corner of the plot). Random guesser is expected to be diagonal and AUC of 0.5. Balanced accuracy point (optimal operating threshold) on ROC curve is the point on the curve for which the sum of sensitivity and specificity is maximal. Sensitivity and specificity of all miRNAs were calculated for this point.

Tab. 7. Receiver Operating Characteristics (ROC) curve parameters for the 9 novel AD miRNA biomarkers and 6 previously reported miRNAs that separate all early and later stage AD patients versus all non-demented controls: AD vs CTR (AD1 and AD2 vs CTR1 and CTR2) and MCI-AD vs CTR (MCI-AD1 and MCI-AD2 vs CTR1 and CTR2). AUC, area under the ROC curve. Analysis was performed with two separate sets of samples called train set (data of the Stage 1, pilot experiment) and test set (data from the Stage 2, verification experiment).

| Novel miRNA biomarkers: early or later AD vs control | | | | | | | | |
|---|---|---|---|---|---|---|---|---|
| | | | Train set | | | Test set | | |
| miRNAs (hsa-miR) | SEQ ID NO | Comparison | AUC | Specificity | Sensitivity | AUC | Specificity | Sensitivity |
| 151a-5p | 1 | AD vs CTR | **0.83** | 1 | 0.57 | **0.90** | 0.77 | 1 |
| 151a-5p | 1 | MCI-AD vs CTR | **0.93** | 1 | 0.71 | **0.79** | 0.88 | 0.87 |
| 30b-5p | 2 | AD vs CTR | **0.95** | 1 | 0.86 | **0.88** | 0.88 | 0.84 |
| 30b-5p | 2 | MCI-AD vs CTR | **0.95** | 0.83 | 1 | **0.82** | 0.89 | 0.87 |
| 486-5p | 3 | AD vs CTR | **0.78** | 1 | 0.57 | **0.93** | 0.89 | 1 |
| 486-5p | 3 | MCI-AD vs CTR | **0.90** | 1 | 0.86 | **0.87** | 0.89 | 0.87 |
| 33a-5p | 4 | AD vs CTR | **0.86** | 1 | 0.86 | **0.78** | 0.78 | 0.85 |
| 33a-5p | 4 | MCI-AD vs CTR | **1** | 1 | 1 | **0.80** | 0.78 | 0.87 |
| 483-5p | 5 | AD vs CTR | **0.92** | 1 | 0.8 | **0.99** | 1 | 0.92 |
| 483-5p | 5 | MCI-AD vs CTR | **0.93** | 1 | 0.83 | **0.95** | 1 | 0.87 |
| 18a-5p | 6 | AD vs CTR | **0.90** | 1 | 0.71 | **0.79** | 0.67 | 0.92 |
| 18a-5p | 6 | MCI-AD vs CTR | **0.95** | 0.83 | 1 | **0.73** | 0.67 | 0.87 |
| 320a | 7 | AD vs CTR | **0.97** | 1 | 0.86 | **0.65** | 0.67 | 0.69 |
| 320a | 7 | MCI-AD vs CTR | **0.93** | 1 | 0.86 | **0.76** | 1 | 0.62 |
| 320b | 8 | AD vs CTR | **0.93** | 1 | 0.86 | **0.83** | 1 | 0.61 |
| 320b | 8 | MCI-AD vs CTR | **0.95** | 1 | 0.86 | **0.79** | 1 | 0.62 |
| 320c | 9 | AD vs CTR | **0.95** | 0.83 | 1 | **0.67** | 1 | 0.38 |
| 320c | 9 | MCI-AD vs CTR | **0.93** | 0.83 | 1 | **0.58** | 0.78 | 0.5 |

(continued)

| Published miRNAs confirmed in the study: early or later AD vs control | | | | | | | | |
|---|---|---|---|---|---|---|---|---|
| 502-3p | 14 | AD vs CTR | **0.90** | 0.83 | 1 | **0.94** | 0.89 | 1 |
| 502-3p | 14 | MCI-AD vs CTR | **0.90** | 0.83 | 0.86 | **0.86** | 0.89 | 0.87 |
| 103a-3p | 15 | AD vs CTR | **0.86** | 0.83 | 0.86 | **0.88** | 0.89 | 0.85 |
| 103a-3p | 15 | MCI-AD vs CTR | **0.88** | 0.83 | 0.86 | **0.76** | 0.89 | 0.75 |
| 301a-3p | 16 | AD vs CTR | **0.86** | 1 | 0.71 | **0.83** | 0.78 | 0.92 |
| 301a-3p | 16 | MCI-AD vs CTR | **0.97** | 1 | 0.86 | **0.80** | 0.78 | 0.87 |
| 142-3p | 17 | AD vs CTR | **0.95** | 1 | 0.86 | **0.79** | 0.89 | 0.77 |
| 142-3p | 17 | MCI-AD vs CTR | **1** | 1 | 1 | **0.80** | 0.78 | 0.87 |
| 200a-3p | 18 | AD vs CTR | **1** | 1 | 1 | **0.90** | 1 | 0.77 |
| 200a-3p | 18 | MCI-AD vs CTR | **1** | 1 | 1 | **0.83** | 0.89 | 0.75 |
| 1260a | 19 | AD vs CTR | **0.88** | 1 | 0.71 | **0.65** | 0.78 | 0.69 |
| 1260a | 19 | MCI-AD vs CTR | **0.81** | 1 | 0.57 | **0.71** | 0.66 | 0.87 |

**Example 3: Functional role of miRNAs in AD pathomechanism**

To further test and validate the suitability of miRNAs in the panel as biomarkers for AD, the inventors functionally mapped the investigated miRNAs to proteins involved in the AD pathomechanism. The 15 biomarker miRNAs showed common (from 6 to 2 miRNAs) functional effectors among proteins directly related to AD pathology, such as APP, BACE, MAPT, PSEN2, as well as other proteins known to contribute to AD, including proteins of the mitochondrial oxidative chain, cell cycle and cell fate kinases MAPK, ERK and JNK, cell cycle and apoptosis regulatory proteins p53 and Bcl-2, insulin signaling IGFRI, autophagy and endocytosis regulatory proteins, cytoskeletal proteins, and proteins of calcium signaling/homeostasis (Tab. 8, Tab. 9, Fig. 8, Fig. 9). This data supports the role of miRNAs in the network regulation and contribution to AD of several signaling pathways, according to the mitochondrial hypothesis, the cell cycle hypothesis, the AD as type 3 diabetes hypothesis, the autophagy/endocytosis hypothesis and other hypothesis postulated by accumulating evidence.

Tab. 8 presents results of an in silico search performed with Targetscan 7.1 software for identifying target mRNAs among the mRNAs encoding microtubule associated protein tau (MAPT) as well as key proteins of the amyloidogenic cleavage of amyloid precursor protein (APP) to toxic amyloid peptide (BACE, PSEN). Toxic amyloid and MAPT are two crucial hallmarks of AD pathology. Importantly, this analysis showed that such crucial AD proteins as APP, BACE 1 and tau (MAPT) can be regulated by more than one miRNA of the investigated 15 miRNAs. Of note, as much as 4 of the 15 miRNAs have binding sites in APP mRNA. Moreover, hsa-miR-483-5p, which we found as one of the most deregulated miRNAs in early AD detection, has a binding site in tau mRNA, together with 4 other miRNAs of the 15 biomarker candidates. Since miRNAs are known to function in complementary regulatory networks, the multiple binding sites in mRNAs closely related to AD pathology for several miRNAs out of the 15 differentiating AD from controls confirms that these miRNAs are relevant in AD pathogenesis.

A group of potential downstream target genes/proteins known to contribute to AD pathogenesis was also identified in an independent approach based on searching the MiRTarBase, comprising experimentally validated miRNA target genes, followed by searching the KEGG database for signaling pathways in neurodegenerative diseases and in the nervous system regulated by the analyzed 15 miRNAs (Tab. 9, Fig. 8). The target proteins were grouped from the ones regulated by 6 miRNAs down to the ones regulated by 2 miRNAs of the 15 miRNAs (Tab. 8, Fig. 9). As shown in Fig. 8, the analysis indicated a network of targets centered around the mitochondria respiratory chain that were implicated in oxidative stress in AD pathology by a vast number of independent reports. MAPK are also known to take center stage in aberrant cellular signaling in AD pathology. Potential downstream effectors regulated by 3 or 2 of the analyzed miRNAs include other proteins known to contribute to AD pathogenesis, such as the insulin growth factor receptor (IGFR I), apoptosis-related proteins such as p53 and Bcl-2, and proteins involved in endocytosis and intracellular signaling (Rab5, ERK) - Fig. 9.

**Tab. 8. Binding sites in AD-related transcripts for the differential 15 miRNAs in AD plasma.** Search was performed with Targetscan 7.1 software. APP- Amyloid Beta Precursor Protein; BACE 1 - Beta-Site Amyloid Precursor Protein Cleaving Enzyme 1 ; BACE 2- Beta-Site Amyloid Precursor Protein Cleaving Enzyme 2; MAPT- Microtubule-Associated Protein Tau Tau Protein ; PSEN2- Presenilin 2.

| Target gene | miRNAs | miRNA and their seed region matching site in the 3'UTR region of the gene |
|---|---|---|
| **APP** | hsa-miR-1260a | hsa-miR-12603 : 80-86 |
| | hsa-miR-320a/b/c | hsa-miR-320a/b/c : 2355-2361 |
| | | 2743-2750 |
| **BACE 1** | hsa-miR-200a-3p | hsa-miR-200a3p : 1551-1558 |
| | hsa-miR-1260a | hsa-miR-1260a : 1559-1565 |
| | hsa-miR-320a/b/c | hsa-miR-320a/b/c : 3216-3222 |
| **BACE 2** | hsa-miR-483-5p | hsa-miR-483-5p : 103-109 |
| | | 3798-3804 |
| **MAPT (Tau)** | hsa-miR-151a-3p | hsa-miR-151a-3p : 101-107 |
| | hsa-miR-483-5p | hsa-miR-4a3-5p : 1143-1149 |
| | hsa-miR-320a/b/c | hsa-miR-320a/b/c : 2795-2801 |
| | | 3885-3891 |
| **PSEN2** | hsa-miR-30b-5p | hsa-miR-30b-5p : 121-127 |

**Tab. 9. Cellular effectors of 15 biomarker miRNAs identified by searching the MirTarBase and the KEGG neurodegenerative diseases and nervous system pathway database.** All target proteins that had at least 2 or more hits in the databases are shown. Several example target genes for only one miRNAs are included.

| Gene/pathway | Hits | Panel miRNAs |
|---|---|---|
| MAPK | 6 | 483-5p, 30b-5p, 33a-5p, 18a-5p, 320b, 320a |
| Cx V | 5 | 483-5p, 151a-5p, 30b-5p, 320b, 320a |
| Cx I | 4 | 483-5p, 30b-5p, 320c, 320a |
| Cx IV | 4 | 18a-5p, 320b, 320a, 320c |
| Sema 7A | 4 | 33a-5p, 320b, 320a, 320c |
| p73 | 3 | 320b, 320a, 320c |
| Dynactin | 3 | 486-5p, 30b-5p, 18a-5p |
| CrK | 3 | 320b, 320a, 320c |
| ERK | 3 | 483-5p, 320b and 320a |
| RSK | 3 | 320b, 320a,320c |
| GLNT | 3 | 320b, 320a,320c |
| p53 | 2 | 30b-5p, 18a-5p |
| IGFIR | 2 | 486-5p, 320a |
| Bcl-2 | 2 | 30b-5p, 18a-5p |
| Rap 1 | 2 | 30b-Sp, 18a-5p |
| JNK | 2 | 30b-5p, 33a-5p |
| Rab 5 | 2 | 33a-5p, 18a-5p |

(continued)

| Gene/pathway | Hits | Panel miRNAs |
|---|---|---|
| Ubc 6/7 | 2 | 18a-5p, 320a |
| Gs | 2 | 18a-5p, 320b |
| GLNS | 2 | 320b, 320a |
| mPTP | 2 | 320a,320c |
| BACE | 1 | 483-5p |
| PARKIN | 1 | 483-5p |
| SOD2 | 1 | 502-3p |
| p38 | 1 | 18a-5p |
| | | |
| GAPD | 1 | 320a |
| CaM | 1 | 320a |
| NFkB | 1 | 320a |

## METHODS

### Study subject characteristics

Blood samples were obtained from patients enrolled in the Alzheimer's Ward of the Central Clinical Hospital of the Ministry of Interior and Administration (MSWiA) in Warsaw. Experimental protocols used for the obtaining and analyzing of blood plasma samples were approved by the Ethics Committee for Studies on Human Subjects at the Central Clinical Hospital of the Ministry of Interior in Warsaw, Poland, and are in compliance with the National and European Union legislation and the Code of Ethical Principles for Medical Research Involving Human Subjects of the World Medical Association. Peripheral blood samples are collected from all subjects after written informed consent was obtained from the patients or their legal representatives.

All clinical diagnoses were performed according to the criteria of the Diagnostic and Statistical Manual of Mental Disorders, 4th edition (DSM-IV), the NINCDS-ADRDA (Criteria of National Institute of Neurological and Communicative Disorders and Stroke and the Alzheimer's Disease and Related Disorders Association) and according to the recommendations from the National Institute of Ageing - Alzheimer's Association workgroups on diagnostic guidelines for Alzheimer's disease [6, 7]. The patients were diagnosed by neurologists, neuropsychologists, and other qualified staff members in the clinic. Diagnosis was based on an interview, the Minimental State Examination (MMSE) test, the Clinical Dementia Rating (CDR) test and radiological tests (MRI). The diagnoses of AD patients recruited for our study were additionally supported by the results of cerebrospinal fluid (CSF) assays based on the levels of the following standard markers: $A\beta$ peptide, t-tau ("total" tau protein) and p-tau (phosphorylated form of tau protein). Patient characteristics are presented in Tab. 10 and 11. The group of patients denoted as AD (AD1 and AD2) comprised 20 patients with clear evidence of developed AD pathological process according to levels of the CSF markers, MMSE results $\geq 11 \leq 26$, and CDR values $\geq 1$ (Tab. 10). The MCI group with a high likelihood that the MCI was due to AD was denoted MCI-AD (MCI-AD1 and MCI-AD2) and comprised 15 patients in early AD stage as confirmed by levels of AD CSF biomarkers, but with lower dementia indicators (MMSE results $\geq 21 \leq 29$ and CDR rating = 0.5). Another group denoted as MCI consisted of 10 patients diagnosed as MCI on the basis of neurological assessment, MMSE results $\geq 24 \leq 30$, CDR = 0.5, but with low probability of AD based on CSF tests. 3 SCI patients were characterized by very mild evidence of dementia: MMSE $\geq 27 \leq 29$ and CDR = 0 and by some probability of early AD based on the slightly positive values of two CSF markers.

The two control groups comprised age-matched subjects without dementia; one group consisted of 6 attendees of the Universities of the Third Age, selected as a group of people at low risk of dementia due to intellectual activity, and not taking any medications. The second control group consisted of 9 patients with hypertension enrolled in the Independent Public Central Clinical Hospital at Banacha st. in Warsaw but without any memory problems and with no family history of AD, who received antihypertensive drugs. Inclusion criteria for the control group are given in Tab. 12.

**Tab.10. Patients characteristics in whole groups enrolled in the study. Mean values +/-SD are shown; t-tau, total tau; p-tau, phosphorylated tau. MMSE- Mini-Mental State Examination, CDR- Clinical Dementia Rating. CSF tests results: Aβ42, amyloid beta 42; t-tau, total tau; p-tau, phosphorylated tau; mean values +/-SD are shown**

| Group | Group size | Age | Sex (f/m) | MMSE | CDR | Aβ42 pg/mL | t-tau pg/mL | p-tau pg/mL |
|---|---|---|---|---|---|---|---|---|
| AD | 20 | 67±8 | 10/10 | 20.4±4.0 | 1.2 | 387.4±162.9 | 774.3±347 | 94.8±38.2 |
| MCI-AD | 15 | 62±6 | 8/7 | 25.9±2.2 | 0.5 | 631.9±351.1 | 444.0±243 | 67.3±30.3 |
| MCI | 10 | 62±5 | 4/6 | 27.5±2.1 | 0.5 | 717.8±116.8 | 199.6±53 | 34.9±7.4 |
| SCI | 3 | 61±4 | 3/0 | 28.3±1.1 | 0 | 821.0±141.3 | 451.7±82 | 67.6±14.1 |

**Tab. 11. Patients characteristics of AD and MCI-AD subject groups enrolled, respectively, in the stage 1 and stage 2 of the study. MMSE- Mini-Mental State Examination, CDR- Clinical Dementia Rating. CSF tests results: Aβ42, amyloid beta 42; t-tau, total tau; p-tau, phosphorylated tau; mean values +/-SD are shown. All patients showed AD-type neuronal injuries in the hippocampus in their MRIs.**

| Group | Size | Age | Sex (f/m) | MMSE | CDR | Aβ42 (pg/ml) | t-tau (pg/ml) | p-tau (pg/ml) |
|---|---|---|---|---|---|---|---|---|
| AD1 | 7 | 72±5 | 3/4 | 19.65±4 | 1 | 380.4±115 | 799.6±268 | 95.1±24 |
| AD2 | 13 | 66±7 | 7/6 | 20.92±4 | 1.4 | 394.4±188 | 749±392 | 94.5±45 |
| MCI-AD1 | 7 | 62±6 | 3/4 | 26.14±2 | 0.5 | 700.4±443 | 355.1±67 | 55.3±13 |
| MCI-AD2 | 8 | 62±6 | 5/3 | 25.62±3 | 0.5 | 563.4±262 | 532.9±312 | 79.3±37 |

**Tab. 12.** Inclusion and exclusion criteria for enrollment in the control groups CTR1 and CTR2

| Inclusion criteria |
|---|
| • Men and women aged 60-70 without dementia |

| Exclusion criteria |
|---|
| • Neurodegenerative diseases (Alzheimer's, Parkinson's, Huntington, FTD, tauopathies, etc.) <br> • Memory impairment: Mild Cognitive Impairment (MCI), subjective cognitive impairment (SCI) <br> • A history of familial Alzheimer's disease <br> • Diabetes type 1 and 2 <br> • Cancer <br> • Schizophrenia <br> • Autism <br> • Depression |

**Blood collection and sample handling**

Blood samples from all study subjects were withdrawn by venipuncture into BD Vacutainer tubes with EDTA and a gel separating plasma from blood cells to avoid hemolysis. The samples were centrifuged immediately. Aliquots of plasma were stored in -80°C. The procedure was carried out in detailed adherence to SOPs (Standard Operating Procedure) established under the international JPND BIOMARKAPD project and accepted by NCI's Early Detection Research Network (EDRN), and in agreement with Exiqon's recommendations.

**RNA isolation**

miRNA isolation from collected blood plasma samples was performed using the miRCURY™ RNA Isolation Kit (Biofluids) according to the manufacturer's recommendation. Briefly, the RNA isolation controls (UniSp2, UniSp4 and UniSp5) were added to the purification step to detect any differences in extraction efficiency. Isolated miRNA was stored at -80°C until use and transported in dry ice.

**Quantitative real time-PCR (qRT-PCR)**

cDNA synthesis and qRT-PCR was performed according to "miRCURY™ microRNA QC PCR Panel - Instruction manual v1.1", as instructed by EXIQON. All miRNAs were reverse transcribed into cDNA in a single reaction step. The cDNA synthesis control (UniSp6) was added in the reverse transcription reaction giving the opportunity to evaluate the RT reaction. cDNA and Exilent SYBR Green mastermix were transferred to the qPCR panel preloaded with primers (EXIQON), using a pipetting robot. Amplification was performed in a Roche Lightcycler 480. Raw Cp values and melting points, as detected by the cycler software, were exported. Reactions with several melting points, or with melting points that are not within assay specifications, were flagged and removed from the data set. Reactions with an amplification efficiency below 1.6 were removed as well. Reactions giving Cp values within 5 Cp values of the negative controls, were removed from the dataset. Moreover, all data were normalized to correct for potential overall differences between the samples. A "no template" sample in the RT step was included as a negative control.

An additional step in the real-time PCR analysis was performed to evaluate the specificity of the amplification products by generating a melting curve for each reaction. A major source of variation in plasma and serum samples is contamination with potential cell-derived miRNA, especially from hemolysis [34]. The hemolysis assessment of the samples in this project showed no sign of red blood cell contamination.

**Bioinformatic and statistical analysis**

Normalization was performed based on the average of the assays detected in all samples as this was shown to be the best normalization for qPCR studies involving numerous assays [35]. For the present study, this included 153 assays. The stability of the average of 153 miRNAs is higher than any single miRNA in the data set as measured by the NormFinder software [36]. The formula used to calculate the normalized Cq values are:

$$\text{Normalized Cq} = \text{average Cq (n)} - \text{assay Cq (sample)}$$

A higher value thus indicates that the miRNA is more abundant in the particular sample.

All groups were compared using a t-test. The Shapiro-Wilk test was used to check whether the data have a normal distribution. When the data were not normally distributed, the Wilcoxon non-parametric statistical hypothesis test was used. A chi-square test was used to identify miRNAs only detectable in one group. To analyze the results of the pilot study, the inventors also used ANOVA. The p-values are shown according to Prism and InStat standards, as follows:

| Symbol | Meaning |
|--------|---------|
| ns | $P > 0.05$ |
| * | $P \leq 0.05$ |
| ** | $P \leq 0.01$ |
| *** | $P \leq 0.001$ |
| **** | $P \leq 0.0001$ |

Receiver operating characteristic (ROC) curves and area under the ROC curve (AUC) values of individual miRNAs were plotted and calculated using the pROC package in R environment as described previously [37]. The specificity and sensitivity values were calculated for the balanced accuracy point (optimal operating threshold) on ROC curves.

**Identification of miRNAs cellular effectors**

Target genes for miRNAs were obtained from two different database analysis. The TargetScan database (release 7.1 ) [38] was used to explore possible/predicted miRNA-mRNA interactions to identify target mRNAs among the mRNAs encoding tau protein and key proteins of the amyloid cascade. In addition, the MirTarbase (version 6.0) database which contains experimentally validated miRNA target genes [39] was used to explore miRNA gene targets which were subsequently mapped to KEGG pathways [40]. Pathways which contribute to neurodegenerative diseases and pathways in the nervous system were analyzed for miRNA targets. The mapping of miRNAs to gene targets in pathways was performed with the R Bioconducor pathview package [41 ]. For the construction of a miRNA regulatory network only target genes/proteins that had 2 or more hits were considered.

**REFERENCES**

1. Alzheimer Disease International, Dementia statistics, http://www.alz.co.uk/research/statistics, accessed November 2015.

2. Hurd MD, Martorell P, Delavande A, Mullen KJ, Langa KM (2013) Monetary Costs of Dementia in the United States. N Engl J Med 368, 1326-34.

3. Jack CR; Knopman DS, Jagust WJ, Peterson RC, Weiner MW, Aisen PS, Shaw LM, Vemuri P, Wiste HJ, Weigand SD, Lesnick TG, Pankratz VS, Donohue MC, Trojanowski JQ (2013) Tracking pathophysiological processes in Alzheimer's disease: An updated hypothetical model of dynamic biomarkers. Lancet Neurol 12, 207-216.

4. Gelosa G, Brooks DJ (2012) The prognostic value of amyloid imaging. Eur J Nucl Med Mol Imaging 39, 1207-1219.

5. Devanand DP, Liu X, Tabert MH, Pradhaban G, Cuasay K, Bell K, de Leon MJ, Doty RL, Stern Y, Pelton GH (2008) Combining early markers strongly predicts conversion from mild cognitive impairment to Alzheimer's disease. Biol Psychiatry 64, 871-879.

6. McKhann GM, Knopman DS, Chertkow H, Hyman BT, Jack CR Jr, Kawas CH, Klunk WE, Koroshetz WJ, Manly JJ, Mayeux R, Mohs RC, Morris JC, Rossor MN, Scheltens P, Carrillo MC, Thies B, Weintraub S, Phelps CH (2011) The diagnosis of dementia due to Alzheimer's disease: recommendations from the National Institute on Aging-Alzheimer's Association workgroups on diagnostic guidelines for Alzheimer's disease. Alzheimers Dement. 7, 263-269.

7. Albert MS, DeKosky ST, Dickson D, Dubois B, Feldman HH, Fox NC, Gamst A, Holtzman DM, Jagust WJ, Petersen RC, Snyder PJ, Carrillo MC, Thies B, Phelps CH (2011) The diagnosis of mild cognitive impairment due to Alzheimer's disease: recommendations from the National Institute on Aging-Alzheimer's Association workgroups on diagnostic guidelines for Alzheimer's disease. Alzheimers Dement. 7, 270-279.

8. Sperling RA, Aisen PS, Beckett LA, Bennett DA, Craft S, Fagan AM, Iwatsubo T, Jack CR Jr, Kaye J, Montine TJ, Park DC, Reiman EM, Rowe CC, Siemers E, Stern Y, Yaffe K, Carrillo MC, Thies B, Morrison-Bogorad M, Wagster MV, Phelps CH (2011) Toward defining the preclinical stages of Alzheimer's disease: recommendations from the National Institute on Aging-Alzheimer's Association workgroups on diagnostic guidelines for Alzheimer's disease. Alzheimers Dement. 7, 280-292.

9. Henriksen K, O'Bryant SE, Hampel H, Trojanowski JQ, Montine TJ, Jeromin A, Blennow K, Lönneborg A, Wyss-Coray T, Soares H, Bazenet C, Sjögren M, Hu W, Lovestone S, Karsdal MA, Weiner MW, and for the Blood-Based Biomarker Interest Group (2014) The future of blood-based biomarkers for Alzheimer's disease. Alzheimer's & Dementia 10, 115-131.

10. Pan Y, Terpstra E, Wang Y, Qiao F, Wang J, Tong Y, Pan B (2016) Dysregulation and Diagnostic Potential of microRNA in Alzheimer's Disease. J Alzheimers Dis 49, 1-12.

11. Wu HZ, Ong KL, Seeher K, J Armstrong N, Thalamuthu A, Brodaty H, Sachdev P, Mather K (2016) Circulating microRNAs as biomarkers of Alzheimer's disease: A systematic review. Journal of Alzheimer's Disease 49, 755-766.

12. Li Y, Qui C, Tu J, Geng B, Yang J, Jiang T, Cui Q (2014) HMDD v2.0: a database for experimentally supported human microRNA and disease associations. Nucleic Acids Research 42, 1070-1074.

13. Koturbash I, Tolleson WH, Guo L, Yu D, Chen S, Hong H, Mattes W, Ning B (2015) microRNAs as pharmacogenomics biomarkers for drug efficacy and drug safety assessment. Biomark. Med. 9, 1153-1176.

14. Mitchell PS, Parkin RK, Kroh EM, Fritz BR, Wyman SK, Pogosova-Agadjanyan EL, Peterson A, Noteboom J, O'Briant KC, Allen A, Lin DW, Urban N, Drescher CW, Knudsen BS, Stirewalt DL, Gentleman R, Vessella RL, Nelson PS, Martin DB, Tewari M (2008) Circulating microRNAs as stable blood-based markers for cancer detection. Proc Natl Acad Sci U S A. 105, 10513-10518.

15. Fabbri M, Paone A, Calore F, Galli R, Gaudio E, Santhanam R, Lovat F, Fadda P, Mao C, Nuovo GJ, Zanesi N, Crawford M, Ozer GH, Wernicke D, Alder H, Caligiuri MA, Nana-Sinkam P, Perrotti D, Croce CM. (2012) MicroRNAs bind to Toll-like receptors to induce prometastatic inflammatory response. Proc Natl Acad Sci U S A 109, E2110-2116.

16. Valadi H, Ekstrom K, Bossios A, Sjostrand M, Lee JJ, Lotvall JO (2007) Exosome-mediated transfer of mRNAs and microRNAs is a novel mechanism of genetic exchange between cells. Nat. Cell Biol. 9, 654-659.

17. Pegtel DM, Cosmopoulos K, Thorley-Lawson DA, Van Eijndhoven MA, Hopmans ES, Lindenberg JL, de Gruijl TD, Würdinger T, Middeldorp JM (2010) Functional delivery of viral miRNAs via exosomes. Proc. Natl. Acad. Sci. U.S.A. 107, 6328-6333.

18. Mittelbrunn M, Gutiérrez-Vázquez C, Villarroya-Beltri C, Gonzalez S, Sanchez-Cabo F, Gonzalez MÁ, Bernad A, Sanchez-Madrid F (2011) Unidirectional transfer of microRNA-loaded exosomes from T cells to antigen-presenting cells. Nat. Commun. 2, 282

19. Montecalvo A, Larregina AT, Shufesky WJ, Stolz DB, Sullivan ML, Karlsson JM, Baty CJ, Gibson GA, Erdos G, Wang Z, Milosevic J, Tkacheva OA, Divito SJ, Jordan R, Lyons-Weiler J, Watkins SC, Morelli AE (2012) Mechanism of transfer of functional microRNAs between mouse dendritic cells via exosomes. Blood 119, 756-766.

20. Geekiyanage H, Jicha GA, Nelson PT, Chan C (2012) Blood serum, miRNA: non-invasive biomarkers for Alzheimer's disease. Experimental neurology 235(2), 491-496.

21. Kumar P, Dezso Z, MacKenzie C, Oestreicher J, Agoulnik S, Byrne M, Bernier F, Yanagimachi M, Aoshima K, Oda, Y (2013) Circulating miRNA biomarkers for Alzheimer's disease. PloS one 8, e69807.

22. Leidinger P, Backes C, Deutscher S, Schmitt K, Mueller SC, Frese K, Haas J, Ruprecht K, Paul F, Stähler C, Lang CJ, Meder B, Bartfai T, Meese E, Keller A (2013) A blood based 12-miRNA signature of Alzheimer disease patients. Genome Biol. 14, R78.

23. Keller A, Backes C, Haas J, Leidinger P, Maetzler W, Deuschle C, Berg D, Ruschil C, Galata V, Ruprecht K, Stähler C (2016) Validating Alzheimer's disease micro RNAs using next-generation sequencing. Alzheimers Dement. doi: 10.1016/j.jalz.2015.12.012.

24. Tan L, Yu JT, Tan MS, Liu QY, Wang HF, Zhang W, Jiang T, Tan L (2014) Genome-wide serum microRNA expression profiling identifies serum biomarkers for Alzheimer's disease. Journal of Alzheimer's Disease 40, 1017-1027.

25. Tan L, Yu JT, Liu QY, Tan MS, Zhang W, Hu N, Wang YL, Sun L, Jiang T, Tan L (2014) Circulating miR-125b as a biomarker of Alzheimer's disease. Journal of the neurological sciences 336, 52-56.

26. Liu CG, Song J, Zhang YQ, Wang PC (2014) MicroRNA-193b is a regulator of amyloid precursor protein in the blood and cerebrospinal fluid derived exosomal microRNA-193b is a biomarker of Alzheimer's disease. Molecular medicine reports 10, 2395-2400.

27. Burgos K, Malenica I, Metpally R, Courtright A, Rakela B, Beach T, Shill H, Adler C, Sabbagh M, Villa S, Tembe W, Craig D, Van Keuren-Jensen K (2014) Profiles of extracellular miRNA in cerebrospinal fluid and serum from patients with Alzheimer's and Parkinson's diseases correlate with disease status and features of pathology. PLoS One 9, e94839.

28. Satoh JI, Kino Y, Niida S (2015) MicroRNA-Seq Data Analysis Pipeline to Identify Blood Biomarkers for Alzheimer's Disease from Public Data. Biomarker insights 10, 21.

29. Lugli G, Cohen AM, Bennett DA, Shah RC, Fields CJ, Hernandez AG, Smalheiser NR (2015) Plasma Exosomal miRNAs in Persons with and without Alzheimer Disease: Altered Expression and Prospects for Biomarkers. PloS one 10, e0139233.

30. Xie B, Zhou H, Zhang R, Song M, Yu L, Wang L, Liu Z, Zhang Q, Cui D, Wang X, Xu S (2015) Serum miR-206 and miR-132 as potential circulating biomarkers for mild cognitive impairment. Journal of Alzheimer's Disease 45, 721-731.

31. Reijs BL, Teunissen CE2, Goncharenko N, Betsou F, Blennow K, Baldeiras I, Brosseron F, Cavedo E, Fladby T, Froelich L, Gabryelewicz T, Gurvit H, Kapaki E, Koson P, Kulic L, Lehmann S, Lewczuk P, Lleó A, Maetzler W, de Mendonça A, Miller AM, Molinuevo JL, Mollenhauer B, Parnetti L, Rot U, Schneider A, Simonsen AH, Tagliavini F, Tsolaki M, Verbeek MM, Verhey FR, Zboch M, Winblad B, Scheltens P, Zetterberg H, Visser PJ (2015) The Central Biobank and Virtual Biobank of BIOMARKAPD: A Resource for Studies on Neurodegenerative Diseases. Front Neurol. 6, 216.

32. O'Bryant SE, Gupta V, Henriksen K, Edwards M, Jeromine A, Lista S, Bazenet C, Soares H, Lovestone S, Hampel H, Montine T, Blennow K, Foroud T, Carrillo M, Graff-Radford N, Laske C, Bretelrq M, Shaw Leslie, Trojanowski JQ, Schupf N, Rissman RA, Fagan AM, Oberoi P, Umek R, Weiner MW, Grammas P, Posner H, Martins R, for the STAR-B and BBBIG working groups (2015) Guidelines for the standardization of preanalytic variables for blood-based biomarker studies in Alzheimer's disease research. Alzheimer's & Dementia 11, 549-560.

33. Lau P, Bossers K, Janky R, Salta E, Frigerio CS, Barbash S, Rothman R, Sierksma AS, Thathiah A, Greenberg D, Papadopoulou AS, Achsel T, Ayoubi T, Soreq H, Verhaagen J, Swaab DF, Aerts S, De Strooper B (2013), Alteration of the microRNA network during the progression of Alzheimer's disease. EMBO Mol Med. 5, 1613-1634.

34. Blondal T, Nielsen SJ, Baker A, Andreasen D, Mouritzen P, Teilum MW, Dahlsveen IK (2013) Assessing sample and miRNA profile quality in serum and plasma or other biofluids. Methods 59, S1-S6.

35. Mestdagh P, Van Vlierberghe P, De Weer A, Muth D, Westermann F, Speleman F, Vandesompele J (2009) A novel and universal method for microRNA RT-qPCR data normalization. Genome Biol. 10, R64.

36. Andersen CL, Jensen JL, Orntoft TF (2004) Normalization of Real-Time Quantitative Reverse Transcription-PCR Data: A Model-Based Variance Estimation Approach to Identify Genes Suited for Normalization, Applied to Bladder and Colon Cancer Data Sets. Cancer Res. 64, 5245-5250.

37. Robin X, Turck N, Hainard A, Tiberti N, Lisacek F, Sanchez JC, Müller M (2011) pROC: an open-source package for R and S+ to analyze and compare ROC curves. BMC Bioinformatics 12, 77.

38. TargetScanHuman: Prediction of microRNA targets. Release 7.1 : June 2016. Agarwal et al., 2015, http://www.targetscan.org/vert_71/, accessed December 2015

39. Chou CH, Chang NW, Shrestha S, Hsu SD, Lin YL, Lee WH, Yang CD, Hong HC, Wei TY, Tu SJ, Tsai TR, Ho SY, Jian TY, Wu HY, Chen PR, Lin NC, Huang HT, Yang TL, Pai CY, Tai CS, Chen WL, Huang CY, Liu CC, Weng SL, Liao KW, Hsu WL, Huang HD. miRTarBase 2016: updates to the experimentally validated miRNA-target interactions database. Nucleic Acids Research. 2016;44:D239-D247.

40. KEGG: Kyoto Encyclopedia of Genes and Genomes, http://www.genome.jp/kegg, accessed July 2016.

41. Luo W, Brouwer C. Pathview: an R/Bioconductor package for pathway-based data integration and visualization. Bioinformatics. 2013;29:1830-1831

**SEQENCE LISTING DESCRIPTION**

| SEQ ID NO: 1. | Human microRNA hsa-miR-151a-5p |
|---|---|
| SEQ ID NO: 2 | Human microRNA hsa-miR-30b-5p |
| SEQ ID NO: 3 | Human microRNA hsa-miR-486-5p |
| SEQ ID NO: 4 | Human microRNA hsa-miR-33a-5p |
| SEQ ID NO: 5 | Human microRNA hsa-miR-483-5p |
| SEQ ID NO: 6 | Human microRNA hsa-miR-18a-5p |
| SEQ ID NO: 7 | Human microRNA hsa-miR-320a |
| SEQ ID NO: 8 | Human microRNA hsa-miR-320b |
| SEQ ID NO: 9 | Human microRNA hsa-miR-320c |
| SEQ ID NO: 10 | Human microRNA hsa-miR-423-5p |
| SEQ ID NO: 11 | Human microRNA hsa-miR-126-5p |
| SEQ ID NO: 12 | Human microRNA hsa-miR-22-5p |
| SEQ ID NO: 13 | Human microRNA hsa-miR-335-3p |
| SEQ ID NO: 14 | Human microRNA hsa-miR-502-3p |
| SEQ ID NO: 15 | Human microRNA hsa-miR-103a-3p |
| SEQ ID NO: 16 | Human microRNA hsa-miR-301a-3p |
| SEQ ID NO: 17 | Human microRNA hsa-miR-142-3p |
| SEQ ID NO: 18 | Human microRNA hsa-miR-200a-3p |
| SEQ ID NO: 19 | Human microRNA hsa-miR-1260a |
| SEQ ID NO: 20 | Human microRNA hsa-miR-185-5p |
| SEQ ID NO: 21 | Human microRNA hsa-miR-128-3p |
| SEQ ID NO: 22 | Human microRNA hsa-miR-130b-3p |
| SEQ ID NO: 23 | Human microRNA hsa-miR-15a-5p |
| SEQ ID NO: 24 | Human microRNA hsa-miR-425-3p |

## Claims

1. A method of *ex vivo* diagnosing Alzheimer's disease and/or an early stage of Alzheimer's disease in a subject, **characterized in that** the method involves the following steps:

   - in vitro assessment of a level of at least hsa-miR-483-5p (SEQ ID NO: 5) and hsa-miR-486-5p (SEQ ID NO: 3) as biomarker microRNAs in the subject's blood sample, and
   - comparison of the assessed level of each biomarker microRNAs with its mean level observed in a non-demented control.

2. Use of a panel of biomarker microRNAs comprising hsa-miR-483-5p (SEQ ID NO: 5) and hsa-miR-486-5p (SEQ ID NO: 3) for diagnosing Alzheimer's disease and/or an early stage of Alzheimer's disease in a subject by analysing the level of said biomarker microRNAs *ex vivo* in a blood sample of said subject.

3. The method of claim 1 or the use of claim 2, wherein additionally a level of at least one additional biomarker microRNA is assessed, wherein the additional biomarker microRNA is selected from the group consisting of hsa-miR-502-3p (SEQ ID NO: 14), hsa-miR-103a-3p (SEQ ID NO: 15), hsa-miR-301 a-3p (SEQ ID NO: 16), hsa-miR-142-3p (SEQ ID NO: 17), hsa-miR-200a-3p (SEQ ID NO: 18), hsa-miR-1260a (SEQ ID NO: 19), wherein preferably the assessed biomarker microRNA(s) include hsa-miR-200a-3p (SEQ ID NO: 18).

4. The method of any one of claims 1 or 3 or the use of any of claims 2 or 3, wherein it involves additionally assessing the level of

   (a) at least one, preferably two, preferably three, preferably four, preferably five, preferably six, preferably all seven biomarker microRNAs selected form a group of hsa-miR-151 a-5p (SEQ ID NO: 1 ), hsa-miR-30b-5p (SEQ ID NO: 2), hsa-miR-33a-5p (SEQ ID NO: 4), hsa-miR-18a-5p (SEQ ID NO: 6), hsa-miR-320a (SEQ ID NO: 7), hsa-miR-320b (SEQ ID NO: 8), hsa-miR-320c (SEQ ID NO: 9), and/or
   (b) hsa-miR-502-3p (SEQ ID NO: 14), hsa-miR-103a-3p (SEQ ID NO: 15), hsa-miR-301a-3p (SEQ ID NO: 16), hsa-miR-142-3p (SEQ ID NO: 17), hsa-miR-200a-3p (SEQ ID NO: 18), and hsa-miR-1260a (SEQ ID NO: 19).

5. The method of any one of claims 1, 3 or 4, or the use of any of claims 2-4, wherein the use or method additionally includes assessment of a level of at least one of biomarker microRNA(s) hsa-miR-423-5p (SEQ ID NO: 10) and/or hsa-miR-126-5p (SEQ ID NO: 11) and/or hsa-miR-22-5p (SEQ ID NO: 12) and/or hsa-miR-335-3p (SEQ ID NO: 13).

6. The method of any one of claims 1, or 3-5, or the use of any of claims 2-5, wherein the blood sample has a volume of at most about 0.6 ml.

7. The method of any one of claims 1, or 3-6, or the use of any of claims 2-6, wherein the assessment of the level of said biomarker microRNA(s) is done by a qRT-PCR method.

8. The method of any one of claims 1, or 3-7, or the use of any of claims 2-7, **characterized in that** the subject is human.

## Patentansprüche

1. Ein Verfahren zur *Ex-vivo*-Diagnose der Alzheimer-Krankheit und/oder eines frühen Stadiums der Alzheimer-Krankheit bei einem Subjekt, **dadurch gekennzeichnet, dass** das Verfahren die folgenden Schritte umfasst:

   - *In-vitro*-Bewertung eines Spiegels von mindestens hsa-miR-483-5p (SEQ ID NO: 5) und hsa-miR-486-5p (SEQ ID NO: 3) als Biomarker-microRNAs in der Blutprobe des Subjekts, und
- Vergleich des bewerteten Spiegels von Biomarker-microRNAs mit ihrem mittleren Spiegel, der bei einer nicht-dementierten Kontrolle beobachtet wurde.

2. Verwendung eines Panels von Biomarker-microRNAs, umfassend hsa-miR-483-5p (SEQ ID NO: 5) und hsa-miR-486-5p (SEQ ID NO: 3) zur Diagnose der Alzheimer-Krankheit und/oder eines frühen Stadiums der Alzheimer-Krankheit in einem Subjekt durch Analyse des Spiegels der Biomarker-microRNAs *ex vivo* in einer Blutprobe des Subjekts.

3. Das Verfahren nach Anspruch 1 oder die Verwendung nach Anspruch 2, wobei zusätzlich ein Spiegel von mindestens einer zusätzlichen Biomarker-microRNA bewertet wird, wobei die zusätzliche Biomarker-microRNA ausgewählt ist aus der Gruppe bestehend aus hsa-miR-502-3p (SEQ ID NO: 14), hsa-miR-103a-3p (SEQ ID NO: 15), hsa-miR-301a-3p (SEQ ID NO: 16), hsa-miR-142-3p (SEQ ID NO: 17), hsa -miR-200a-3p (SEQ ID NO: 18), hsa-miR-1260a (SEQ ID NO: 19), wobei vorzugsweise die bewerteten Biomarker-microRNA(s) hsa-miR-200a-3p (SEQ ID NO: 18) umfassen.

4. Das Verfahren nach einem der Ansprüche 1 oder 3 oder die Verwendung nach einem der Ansprüche 2 oder 3, wobei es/sie zusätzlich das Bewerten des Spiegels von

(a) mindestens einer, vorzugsweise zwei, vorzugsweise drei, vorzugsweise vier, vorzugsweise fünf, vorzugsweise sechs, vorzugsweise alle sieben Biomarker-microRNAs, ausgewählt aus einer Gruppe von hsa-miR-151 a-5p (SEQ ID NO: 1), hsa- miR-30b-5p (SEQ ID NO: 2), hsa-miR-33a-5p (SEQ ID NO: 4), hsa-miR-18a-5p (SEQ ID NO: 6), hsa-miR-320a (SEQ ID NO: 7), hsa-miR-320b (SEQ ID NO: 8), hsa-miR-320c (SEQ ID NO: 9), und/oder
(b) hsa-miR-502-3p (SEQ ID NO: 14), hsa-miR-103a-3p (SEQ ID NO: 15), hsa-miR-301a-3p (SEQ ID NO: 16), hsa -miR-142-3p (SEQ ID NO: 17), hsa-miR-200a-3p (SEQ ID NO: 18), und hsa-miR-1260a (SEQ ID NO: 19),

umfasst.

5. Das Verfahren nach einem der Ansprüche 1, 3 oder 4, oder die Verwendung nach einem der Ansprüche 2-4, wobei die Verwendung oder das Verfahren zusätzlich eine Bewertung eines Spiegels von mindestens einer der Biomarker-microRNA(s) hsa-miR-423-5p (SEQ ID NO: 10) und/oder hsa-miR-126-5p (SEQ ID NO: 11) und/oder hsa-miR-22-5p (SEQ ID NO: 12) und/oder hsa-miR -335-3p (SEQ ID NO: 13) umfasst.

6. Das Verfahren nach einem der Ansprüche 1, oder 3-5, oder die Verwendung nach einem der Ansprüche 2-5, wobei die Blutprobe ein Volumen von höchstens etwa 0,6 ml aufweist.

7. Das Verfahren nach einem der Ansprüche 1, oder 3-6, oder die Verwendung nach einem der Ansprüche 2-6, wobei die Bewertung des Spiegels von der Biomarker-microRNA(s) durch ein qRT-PCR-Verfahren erfolgt.

8. Das Verfahren nach einem der Ansprüche 1, oder 3-7, oder die Verwendung nach einem der Ansprüche 2-7, **gekennzeichnet dadurch, dass** das Subjekt ein Mensch ist.


**Revendications**

1. Une méthode de diagnostic *ex vivo* de la maladie d'Alzheimer et/ou d'un stade précoce de la maladie d'Alzheimer chez un sujet, **caractérisée en ce que** la méthode comprend les étapes suivantes:

- évaluation in vitro du niveau d'au moins hsa-miR-483-5p (SEQ ID NO: 5) et hsa-miR-486-5p (SEQ ID NO: 3) en tant que micro-ARN biomarqueurs dans un échantillon de sang du sujet, et
- comparaison du niveau évalué de chaque microARN biomarqueur avec son niveau moyen observé chez un témoin non dément.

2. Utilisation d'un panel de micro-ARN biomarqueurs comprenant hsa-miR-483-5p (SEQ ID NO: 5) et hsa-miR-486-5p (SEQ ID NO: 3) dans le diagnostic de la maladie d'Alzheimer et/ou d'un stade précoce de la maladie d'Alzheimer chez un sujet en analysant le niveau desdits micro-ARN biomarqueurs *ex vivo* dans un échantillon de sang dudit sujet.

**3.** La méthode selon la revendication 1 ou l'utilisation selon la revendication 2, où en outre un niveau d'au moins un micro-ARN biomarqueur supplémentaire est évalué, où le micro-ARN biomarqueur supplémentaire est sélectionné dans le groupe constitué de hsa-miR-502-3p (SEQ ID NO: 14), hsa-miR-103a-3p (SEQ ID NO: 15), hsa-miR-301 a-3p (SEQ ID NO: 16), hsa-miR-142-3p (SEQ ID NO: 17), hsa -miR-200a-3p (SEQ ID NO: 18), hsa-miR-1260a (SEQ ID NO: 19), où de préférence le(s) micro-ARN biomarqueur(s) évalué(s) comprend/comprennent hsa-miR-200a-3p (SEQ ID NO: 18).

**4.** La méthode selon l'une quelconque des revendications 1 ou 3 ou l'utilisation selon l'une quelconque des revendications 2 ou 3, où elle comprend en outre l'évaluation du niveau de

(a) au moins un, de préférence deux, de préférence trois, de préférence quatre, de préférence cinq, de préférence six, de préférence sept micro-ARN biomarqueurs sélectionnés dans le groupe de hsa-miR-151 a-5p (SEQ ID NO: 1), hsa- miR-30b-5p (SEQ ID NO: 2), hsa-miR-33a-5p (SEQ ID NO: 4), hsa-miR-18a-5p (SEQ ID NO: 6), hsa-miR-320a (SEQ ID NO: 7), hsa-miR-320b (SEQ ID NO: 8), hsa-miR-320c (SEQ ID NO: 9), et/ou
(b) hsa-miR-502-3p (SEQ ID NO: 14), hsa-miR-103a-3p (SEQ ID NO: 15), hsa-miR-301a-3p (SEQ ID NO: 16), hsa- miR-142-3p (SEQ ID NO: 17), hsa-miR-200a-3p (SEQ ID NO: 18), et hsa-miR-1260a (SEQ ID NO: 19).

**5.** La méthode selon l'une quelconque des revendications 1, 3 ou 4, ou l'utilisation selon l'une quelconque des revendications 2-4, où l'utilisation ou la méthode comprend en outre l'évaluation du niveau d'au moins l'un des micro-ARN biomarqueur(s) hsa-miR-423-5p (SEQ ID NO: 10) et/ou hsa-miR-126-5p (SEQ ID NO: 11) et/ou hsa-miR-22-5p (SEQ ID NO 12) et/ou hsa-miR-335-3p (SEQ ID NO: 13).

**6.** La méthode selon l'une quelconque des revendications 1 ou 3-5, ou l'utilisation selon l'une quelconque des revendications 2-5, où l'échantillon de sang a un volume d'au plus environ 0,6 ml.

**7.** La méthode selon l'une quelconque des revendications 1 ou 3-6, ou l'utilisation selon l'une quelconque des revendications 2-6, où l'évaluation du niveau desdits micro-ARN biomarqueur(s) est effectuée par une méthode qRT-PCR.

**8.** La méthode selon l'une quelconque des revendications 1 ou 3-7, ou l'utilisation selon l'une quelconque des revendications 2-7, **caractérisé en ce que** le sujet est humain.

Sample collection

- Recruitment of patients and control subjects according to inclusion/exclusion criteria
- Collection of plasma samples

Stage 1
pilot experiment

- Isolation of miRNA from 20 subjects
- microRNA qRT-PCR for 178 miRNAs
- Data collection and quality control
- Negative control evaluation
- Normalization
- Data analysis I

Stage 2
verification experiment

- Isolation of miRNA from 40 subjects
- microRNA qRT-PCR for 15 miRNAs
- Data collection an quality control
- Negative control evaluation
- Normalization
- Data analysis II

**Fig. 1.**

**A.**

**B.**

**Fig. 2**

C.

| | hsa-miR-151a-5p | hsa-miR-30b-5p | hsa-miR-486-5p | hsa-miR-33a-5p | hsa-miR-483-5p | hsa-miR-18a-5p | hsa-miR-320a | hsa-miR-320b | hsa-miR-320c | hsa-miR-502-3p | hsa-miR-103a-3p | hsa-miR-301a-3p | hsa-miR-142-3p | hsa-miR-200a-3p | hsa-miR-1260a |
|---|---|---|---|---|---|---|---|---|---|---|---|---|---|---|---|
| ■ AD1 vs CTR1 | -1,48 | -1,5 | 1,669 | -2,03 | 4,723 | -1,45 | 1,553 | 1,723 | 2,035 | 1,786 | -1,29 | -1,76 | -1,97 | 5,267 | 2,009 |
| ▨ MCI-AD1 vs CTR1 | -1,55 | -1,46 | 1,723 | -1,73 | 5,589 | -1,43 | 1,342 | 1,551 | 1,704 | 1,654 | -1,33 | -1,48 | -1,8 | 4 | 1,761 |

Fig. 2.- continuation

**AD1 vs MCI-AD1**

| | hsa-miR-423-5p | hsa-miR-126-5p | hsa-miR-22-5p | hsa-miR-335-3p |
|---|---|---|---|---|
| ■ AD1 vs MCI-AD1 | 1,2 | 1,25 | 1,22 | -1,99 |

Fig. 3

**MCI-AD1 vs AD1**

Legend:
- ⋯ hsa–miR–22–5p
- – – hsa–miR–126–5p
- –·– hsa–miR–335–3p
- — hsa–miR–423–5p

**Fig. 4.**

**A.**

**AD2 vs CTR2**

| | hsa-miR-151a-5p | hsa-miR-30b-5p | hsa-miR-486-5p | hsa-miR-33a-5p | hsa-miR-483-5p | hsa-miR-18a-5p | hsa-miR-320a | hsa-miR-320b | hsa-miR-320c | hsa-miR-502-3p | hsa-miR-103a-3p | hsa-miR-301a-3p | hsa-miR-142-3p | hsa-miR-200a-3p | hsa-miR-1260a |
|---|---|---|---|---|---|---|---|---|---|---|---|---|---|---|---|
| ■ AD2 vs CTR2 | -2,17 | -1,93 | 5,11 | -1,82 | 13,45 | -1,27 | 1,157 | 1,262 | 1,171 | 2,701 | -1,62 | -1,59 | -1,5 | 3,15 | 1,094 |

**B.**

**MCI-AD2 vs CTR2**

| | hsa-miR-151a-5p | hsa-miR-30b-5p | hsa-miR-486-5p | hsa-miR-33a-5p | hsa-miR-483-5p | hsa-miR-18a-5p | hsa-miR-320a | hsa-miR-320b | hsa-miR-320c | hsa-miR-502-3p | hsa-miR-103a-3p | hsa-miR-301a-3p | hsa-miR-142-3p | hsa-miR-200a-3p | hsa-miR-1260a |
|---|---|---|---|---|---|---|---|---|---|---|---|---|---|---|---|
| ▨ MCI-AD2 vs CTR2 | -1,49 | -1,53 | 3,959 | -1,34 | 9,91 | -1,21 | 1,143 | 1,162 | -1,02 | 2,213 | -1,28 | -1,52 | -1,32 | 2,702 | 1,044 |

**Fig. 5.**

**C.**

## MCI vs CTR2

| | hsa-miR-151a-5p | hsa-miR-30b-5p | hsa-miR-486-5p | hsa-miR-33a-5p | hsa-miR-483-5p | hsa-miR-18a-5p | hsa-miR-320a | hsa-miR-320b | hsa-miR-320c | hsa-miR-502-3p | hsa-miR-103a-3p | hsa-miR-301a-3p | hsa-miR-142-3p | hsa-miR-200a-3p | hsa-miR-1260a |
|---|---|---|---|---|---|---|---|---|---|---|---|---|---|---|---|
| ▦ MCI vs CTR2 | -2,26 | -2,03 | 5,122 | -2,11 | 7,809 | -1,24 | 1,189 | 1,195 | -1,02 | 2,365 | -1,58 | -1,64 | -1,57 | 2,418 | -1,17 |

**D.**

## SCI vs CTR2

| | hsa-miR-151a-5p | hsa-miR-30b-5p | hsa-miR-486-5p | hsa-miR-33a-5p | hsa-miR-483-5p | hsa-miR-18a-5p | hsa-miR-320a | hsa-miR-320b | hsa-miR-320c | hsa-miR-502-3p | hsa-miR-103a-3p | hsa-miR-301a-3p | hsa-miR-142-3p | hsa-miR-200a-3p | hsa-miR-1260a |
|---|---|---|---|---|---|---|---|---|---|---|---|---|---|---|---|
| ▦ SCI vs CTR2 | 1,039 | -1,1 | 5,103 | -2,94 | 11,64 | -1 | 1,593 | 1,518 | 1,26 | 2,097 | 1,049 | -1,08 | -1,06 | 2,585 | 1,585 |

## Fig. 5.- continuation

| | hsa-miR-151a-5p | hsa-miR-30b-5p | hsa-miR-486-5p | hsa-miR-33a-5p | hsa-miR-483-5p | hsa-miR-18a-5p | hsa-miR-320a | hsa-miR-320b | hsa-miR-320c | hsa-miR-502-3p | hsa-miR-103a-3p | hsa-miR-301a-3p | hsa-miR-142-3p | hsa-miR-200a-3p | hsa-miR-1260a |
|---|---|---|---|---|---|---|---|---|---|---|---|---|---|---|---|
| ▉ AD2 vs CTR2 | -2,17 | -1,93 | 5,11 | -1,82 | 13,45 | -1,27 | 1,157 | 1,262 | 1,171 | 2,701 | -1,62 | -1,59 | -1,5 | 3,15 | 1,094 |
| ▨ MCI-AD2 vs CTR2 | -1,49 | -1,53 | 3,959 | -1,34 | 9,91 | -1,21 | 1,143 | 1,162 | -1,02 | 2,213 | -1,28 | -1,52 | -1,32 | 2,702 | 1,044 |
| ▤ MCI vs CTR2 | -2,26 | -2,03 | 5,122 | -2,11 | 7,809 | -1,24 | 1,189 | 1,195 | -1,02 | 2,365 | -1,58 | -1,64 | -1,57 | 2,418 | -1,17 |
| ▉ SCI vs CTR2 | 1,039 | -1,1 | 5,103 | -2,94 | 11,64 | -1 | 1,593 | 1,518 | 1,26 | 2,097 | 1,049 | -1,08 | -1,06 | 2,585 | 1,585 |

## Fig. 6.

Fig. 7.

Fig. 8 (A)

Fig. 8 (B)

**Fig. 9.**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- PL 416956

- PL IB2016052440 W

### Non-patent literature cited in the description

- Diagnostic and Statistical Manual of Mental Disorders. NINCDS-ADRDA (Criteria of National Institute of Neurological and Communicative Disorders and Stroke and the Alzheimer's Disease and Related Disorders Association)
- Dementia statistics. *Alzheimer Disease International,* November 2015, http://www.alz.co.uk/research/statistics
- HURD MD ; MARTORELL P ; DELAVANDE A ; MULLEN KJ ; LANGA KM. Monetary Costs of Dementia in the United States. *N Engl J Med,* 2013, vol. 368, 1326-34
- JACK CR ; KNOPMAN DS ; JAGUST WJ ; PETERSON RC ; WEINER MW ; AISEN PS ; SHAW LM ; VEMURI P ; WISTE HJ ; WEIGAND SD. Tracking pathophysiological processes in Alzheimer's disease: An updated hypothetical model of dynamic biomarkers. *Lancet Neurol,* 2013, vol. 12, 207-216
- GELOSA G ; BROOKS DJ. The prognostic value of amyloid imaging. *Eur J Nucl Med Mol Imaging,* 2012, vol. 39, 1207-1219
- DEVANAND DP ; LIU X ; TABERT MH ; PRADHABAN G ; CUASAY K ; BELL K ; DE LEON MJ ; DOTY RL ; STERN Y ; PELTON GH. Combining early markers strongly predicts conversion from mild cognitive impairment to Alzheimer's disease. *Biol Psychiatry,* 2008, vol. 64, 871-879
- MCKHANN GM ; KNOPMAN DS ; CHERTKOW H ; HYMAN BT ; JACK CR JR ; KAWAS CH ; KLUNK WE ; KOROSHETZ WJ ; MANLY JJ ; MAYEUX R. The diagnosis of dementia due to Alzheimer's disease: recommendations from the National Institute on Aging-Alzheimer's Association workgroups on diagnostic guidelines for Alzheimer's disease. *Alzheimers Dement,* 2011, vol. 7, 263-269

- ALBERT MS ; DEKOSKY ST ; DICKSON D ; DUBOIS B ; FELDMAN HH ; FOX NC ; GAMST A ; HOLTZMAN DM ; JAGUST WJ ; PETERSEN RC. The diagnosis of mild cognitive impairment due to Alzheimer's disease: recommendations from the National Institute on Aging-Alzheimer's Association workgroups on diagnostic guidelines for Alzheimer's disease. *Alzheimers Dement,* 2011, vol. 7, 270-279
- SPERLING RA ; AISEN PS ; BECKETT LA ; BENNETT DA ; CRAFT S ; FAGAN AM ; IWATSUBO T ; JACK CR JR ; KAYE J ; MONTINE TJ. Toward defining the preclinical stages of Alzheimer's disease: recommendations from the National Institute on Aging-Alzheimer's Association workgroups on diagnostic guidelines for Alzheimer's disease. *Alzheimers Dement,* 2011, vol. 7, 280-292
- HENRIKSEN K ; O'BRYANT SE ; HAMPEL H ; TROJANOWSKI JQ ; MONTINE TJ ; JEROMIN A ; BLENNOW K ; LÖNNEBORG A ; WYSS-CORAY T ; SOARES H. Blood-Based Biomarker Interest Group (2014) The future of blood-based biomarkers for Alzheimer's disease. *Alzheimer's & Dementia,* 2014, vol. 10, 115-131
- PAN Y ; TERPSTRA E ; WANG Y ; QIAO F ; WANG J ; TONG Y ; PAN B. Dysregulation and Diagnostic Potential of microRNA in Alzheimer's Disease. *J Alzheimers Dis,* 2016, vol. 49, 1-12
- WU HZ ; ONG KL ; SEEHER K ; J ARMSTRONG N ; THALAMUTHU A ; BRODATY H ; SACHDEV P ; MATHER K. Circulating microRNAs as biomarkers of Alzheimer's disease: A systematic review. *Journal of Alzheimer's Disease,* 2016, vol. 49, 755-766
- LI Y ; QUI C ; TU J ; GENG B ; YANG J ; JIANG T ; CUI Q. HMDD v2.0: a database for experimentally supported human microRNA and disease associations. *Nucleic Acids Research,* 2014, vol. 42, 1070-1074
- KOTURBASH I ; TOLLESON WH ; GUO L ; YU D ; CHEN S ; HONG H ; MATTES W ; NING B. microRNAs as pharmacogenomics biomarkers for drug efficacy and drug safety assessment. *Biomark. Med.,* 2015, vol. 9, 1153-1176

- **MITCHELL PS ; PARKIN RK ; KROH EM ; FRITZ BR ; WYMAN SK ; POGOSOVA-AGADJANYAN EL ; PETERSON A ; NOTEBOOM J ; O'BRIANT KC ; ALLEN A.** Circulating microRNAs as stable blood-based markers for cancer detection. *Proc Natl Acad Sci U S A.,* 2008, vol. 105, 10513-10518

- **FABBRI M ; PAONE A ; CALORE F ; GALLI R ; GAUDIO E ; SANTHANAM R ; LOVAT F ; FADDA P ; MAO C ; NUOVO GJ.** MicroRNAs bind to Toll-like receptors to induce prometastatic inflammatory response. *Proc Natl Acad Sci U S A,* 2012, vol. 109, E2110-2116

- **VALADI H ; EKSTROM K ; BOSSIOS A ; SJOSTRAND M ; LEE JJ ; LOTVALL JO.** Exosome-mediated transfer of mRNAs and microRNAs is a novel mechanism of genetic exchange between cells. *Nat. Cell Biol.,* 2007, vol. 9, 654-659

- **PEGTEL DM ; COSMOPOULOS K ; THORLEY-LAWSON DA ; VAN EIJNDHOVEN MA ; HOPMANS ES ; LINDENBERG JL ; DE GRUIJL TD ; WÜRDINGER T ; MIDDELDORP JM.** Functional delivery of viral miRNAs via exosomes. *Proc. Natl. Acad. Sci. U.S.A.,* 2010, vol. 107, 6328-6333

- **MITTELBRUNN M ; GUTIÉRREZ-VÁZQUEZ C ; VILLARROYA-BELTRI C ; GONZALEZ S ; SANCHEZ-CABO F ; GONZALEZ MÁ ; BERNAD A ; SANCHEZ-MADRID F.** Unidirectional transfer of microRNA-loaded exosomes from T cells to antigen-presenting cells. *Nat. Commun.,* 2011, vol. 2, 282

- **MONTECALVO A ; LARREGINA AT ; SHUFESKY WJ ; STOLZ DB ; SULLIVAN ML ; KARLSSON JM ; BATY CJ ; GIBSON GA ; ERDOS G ; WANG Z.** Mechanism of transfer of functional microRNAs between mouse dendritic cells via exosomes. *Blood,* 2012, vol. 119, 756-766

- **GEEKIYANAGE H ; JICHA GA ; NELSON PT ; CHAN C.** Blood serum, miRNA: non-invasive biomarkers for Alzheimer's disease. *Experimental neurology,* 2012, vol. 235 (2), 491-496

- **KUMAR P ; DEZSO Z ; MACKENZIE C ; OESTREICHER J ; AGOULNIK S ; BYRNE M ; BERNIER F ; YANAGIMACHI M ; AOSHIMA K ; ODA, Y.** Circulating miRNA biomarkers for Alzheimer's disease. *PloS one,* 2013, vol. 8, e69807

- **LEIDINGER P ; BACKES C ; DEUTSCHER S ; SCHMITT K ; MUELLER SC ; FRESE K ; HAAS J ; RUPRECHT K ; PAUL F ; STÄHLER C.** A blood based 12-miRNA signature of Alzheimer disease patients. *Genome Biol.,* 2013, vol. 14, R78

- **KELLER A ; BACKES C ; HAAS J ; LEIDINGER P ; MAETZLER W ; DEUSCHLE C ; BERG D ; RUSCHIL C ; GALATA V ; RUPRECHT K.** Validating Alzheimer's disease micro RNAs using next-generation sequencing. *Alzheimers Dement,* 2016

- **TAN L ; YU JT ; TAN MS ; LIU QY ; WANG HF ; ZHANG W ; JIANG T ; TAN L.** Genome-wide serum microRNA expression profiling identifies serum biomarkers for Alzheimer's disease. *Journal of Alzheimer's Disease,* 2014, vol. 40, 1017-1027

- **TAN L ; YU JT ; LIU QY ; TAN MS ; ZHANG W ; HU N ; WANG YL ; SUN L ; JIANG T ; TAN L.** Circulating miR-125b as a biomarker of Alzheimer's disease. *Journal of the neurological sciences,* 2014, vol. 336, 52-56

- **LIU CG ; SONG J ; ZHANG YQ ; WANG PC.** MicroRNA-193b is a regulator of amyloid precursor protein in the blood and cerebrospinal fluid derived exosomal microRNA-193b is a biomarker of Alzheimer's disease. *Molecular medicine reports,* 2014, vol. 10, 2395-2400

- **BURGOS K ; MALENICA I ; METPALLY R ; COURTRIGHT A ; RAKELA B ; BEACH T ; SHILL H ; ADLER C ; SABBAGH M ; VILLA S.** Profiles of extracellular miRNA in cerebrospinal fluid and serum from patients with Alzheimer's and Parkinson's diseases correlate with disease status and features of pathology. *PLoS One,* 2014, vol. 9, e94839

- **SATOH JI ; KINO Y ; NIIDA S.** MicroRNA-Seq Data Analysis Pipeline to Identify Blood Biomarkers for Alzheimer's Disease from Public Data. *Biomarker insights,* 2015, vol. 10, 21

- **LUGLI G ; COHEN AM ; BENNETT DA ; SHAH RC ; FIELDS CJ ; HERNANDEZ AG ; SMALHEISER NR.** Plasma Exosomal miRNAs in Persons with and without Alzheimer Disease: Altered Expression and Prospects for Biomarkers. *PloS one,* 2015, vol. 10, e0139233

- **XIE B ; ZHOU H ; ZHANG R ; SONG M ; YU L ; WANG L ; LIU Z ; ZHANG Q ; CUI D ; WANG X.** Serum miR-206 and miR-132 as potential circulating biomarkers for mild cognitive impairment. *Journal of Alzheimer's Disease,* 2015, vol. 45, 721-731

- **REIJS BL ; TEUNISSEN CE2 ; GONCHARENKO N ; BETSOU F ; BLENNOW K ; BALDEIRAS I ; BROSSERON F ; CAVEDO E ; FLADBY T ; FROELICH L.** The Central Biobank and Virtual Biobank of BIOMARKAPD: A Resource for Studies on Neurodegenerative Diseases. *Front Neurol,* 2015, vol. 6, 216

- **O'BRYANT SE ; GUPTA V ; HENRIKSEN K ; EDWARDS M ; JEROMINE A ; LISTA S ; BAZENET C ; SOARES H ; LOVESTONE S ; HAMPEL H.** for the STAR-B and BBBIG working groups (2015) Guidelines for the standardization of preanalytic variables for blood-based biomarker studies in Alzheimer's disease research. *Alzheimer's & Dementia,* vol. 11, 549-560

- **LAU P ; BOSSERS K ; JANKY R ; SALTA E ; FRIGERIO CS ; BARBASH S ; ROTHMAN R ; SIERKSMA AS ; THATHIAH A ; GREENBERG D.** Alteration of the microRNA network during the progression of Alzheimer's disease. *EMBO Mol Med.,* 2013, vol. 5, 1613-1634
- **BLONDAL T ; NIELSEN SJ ; BAKER A ; ANDREASEN D ; MOURITZEN P ; TEILUM MW ; DAHLSVEEN IK.** Assessing sample and miRNA profile quality in serum and plasma or other biofluids. *Methods,* 2013, vol. 59, S1-S6
- **MESTDAGH P ; VAN VLIERBERGHE P ; DE WEER A ; MUTH D ; WESTERMANN F ; SPELEMAN F ; VANDESOMPELE J.** A novel and universal method for microRNA RT-qPCR data normalization. *Genome Biol.,* 2009, vol. 10, R64
- **ANDERSEN CL ; JENSEN JL ; ORNTOFT TF.** Normalization of Real-Time Quantitative Reverse Transcription-PCR Data: A Model-Based Variance Estimation Approach to Identify Genes Suited for Normalization, Applied to Bladder and Colon Cancer Data Sets. *Cancer Res.,* 2004, vol. 64, 5245-5250
- **ROBIN X ; TURCK N ; HAINARD A ; TIBERTI N ; LISACEK F ; SANCHEZ JC ; MÜLLER M.** pROC: an open-source package for R and S+ to analyze and compare ROC curves. *BMC Bioinformatics,* 2011, vol. 12, 77
- **AGARWAL et al.** *TargetScanHuman: Prediction of microRNA targets,* December 2015, http://www.targetscan.org/vert_71
- **CHOU CH ; CHANG NW ; SHRESTHA S ; HSU SD ; LIN YL ; LEE WH ; YANG CD ; HONG HC ; WEI TY ; TU SJ.** miRTarBase 2016: updates to the experimentally validated miRNA-target interactions database. *Nucleic Acids Research,* 2016, vol. 44, D239-D247
- *KEGG: Kyoto Encyclopedia of Genes and Genomes,* July 2016, http://www.genome.jp/kegg
- **LUO W ; BROUWER C.** Pathview: an R/Bioconductor package for pathway-based data integration and visualization. *Bioinformatics,* 2013, vol. 29, 1830-1831

SEQUENCE LISTING

<110> Biotech Innovations Sp. z .o. o. Instytut Biologii Dowiadczalnej im. Marcelego Nenckiego Polska Akademia Nauk

<120> PPanel of microRNA biomarkers in blood for diagnosis of Alzheimer's disease

<130> 195371

<150> PL416956
<151> 2016-04-25

<150> PCT/IB2016/052440
<151> 2016-04-29

<160> 24

<170> PatentIn version 3.5

<210> 1
<211> 21
<212> RNA
<213> Homo sapiens

<400> 1
ucgaggagcu cacagucuag u          21

<210> 2
<211> 22
<212> RNA
<213> Homo sapiens

<400> 2
uguaaacauc cuacacucag cu          22

<210> 3
<211> 22
<212> RNA
<213> Homo sapiens

<400> 3
uccuguacug agcugccccg ag          22

<210> 4
<211> 21
<212> RNA
<213> Homo sapiens

<400> 4
gugcauugua guugcauugc a           21

<210> 5
<211> 22
<212> RNA
<213> Homo sapiens

<400> 5
aagacgggag gaaagaaggg ag          22

<210> 6
<211> 23
<212> RNA
<213> Homo sapiens

<400> 6
uaaggugcau cuagugcaga uag          23

<210> 7
<211> 22
<212> RNA
<213> Homo sapiens

<400> 7
aaaagcuggg uugagagggc ga          22

<210> 8
<211> 22
<212> RNA
<213> Homo sapiens

<400> 8
aaaagcuggg uugagagggc aa          22

<210> 9
<211> 20

<212> RNA
<213> Homo sapiens

<400> 9
aaaagcuggg uugagagggu          20

<210> 10
<211> 23
<212> RNA
<213> Homo sapiens

<400> 10
ugaggggcag agagcgagac uuu          23

<210> 11
<211> 21
<212> RNA
<213> Homo sapiens

<400> 11
cauuauuacu uuugguacgc g          21

<210> 12
<211> 22
<212> RNA
<213> Homo sapiens

<400> 12
aguucuucag uggcaagcuu ua          22

<210> 13
<211> 22
<212> RNA
<213> Homo sapiens

<400> 13
uuuuucauua uugcuccuga cc          22

<210> 14
<211> 22
<212> RNA
<213> Homo sapiens

<400> 14
aaugcaccug ggcaaggauu ca          22

<210> 15
<211> 23
<212> RNA
<213> Homo sapiens

<400> 15
agcagcauug uacagggcua uga          23

<210> 16
<211> 23
<212> RNA
<213> Homo sapiens

<400> 16
cagugcaaua guauugucaa age        23


<210> 17
<211> 23
<212> RNA
<213> Homo sapiens


<400> 17
uguaguguuu ccuacuuuau gga        23


<210> 18
<211> 22
<212> RNA
<213> Homo sapiens


<400> 18
uaacacuguc ugguaacgau gu        22


<210> 19
<211> 18
<212> RNA
<213> Homo sapiens


<400> 19
aucccaccuc ugccacca        18


<210> 20
<211> 22
<212> RNA
<213> Homo sapiens


<400> 20
uggagagaaa ggcaguuccu ga        22


<210> 21
<211> 21
<212> RNA
<213> Homo sapiens


<400> 21
ucacagugaa ccggucucuu u        21


<210> 22
<211> 22
<212> RNA
<213> Homo sapiens


<400> 22
cagugcaaug augaaagggc au        22


<210> 23
<211> 22
<212> RNA
<213> Homo sapiens


<400> 23
uagcagcaca uaaugguuug ug        22

<210> 24
<211> 22
<212> RNA
<213> Homo sapiens

<400> 24
aucgggaaug ucguguccgc cc      22